# EUROPEAN PATENT APPLICATION

(11) **EP 4 748 819 A1**
(43) Date of publication of application: **27.05.2026**
(21) Application number: 24215484.7
(22) Date of filing: 26.11.2024
(51) Int. Cl.: C07C 29/34, C07C 31/12, C07C 51/09, C07C 53/08, C07C 67/00, C07C 69/14

(54) **A PROCESS FOR PRODUCING AN ACID**

(71) Applicant: BASF SE, 67056 Ludwigshafen am Rhein (DE)
(72) Inventor: The designation of the inventor has not yet been filed
(74) Representative: Altmann Stößel Dick Patentanwälte PartG mbB

(57) **Abstract**

The present invention relates to a process for producing an acid, and in particular to an alcohol conversion process employing a homogeneous catalyst and a base, wherein an ester as a byproduct of said alcohol conversion process is formed, and wherein an acid from the ester is obtained.

## Description

The present invention relates to a process for producing an acid, and in particular to an alcohol conversion process employing a homogeneous catalyst and a base, wherein an ester as a byproduct of said alcohol conversion process is formed, and wherein an acid from the ester is obtained.

A commonly used industrial production of alcohols is mainly based on an oxo process. Said process comprises the reaction of an alkene with oxo gas, which is a mixture of hydrogen and carbon monoxide in a 1:1 molar ratio. The reaction is followed by hydrogenation of the aldehyde into the desired alcohol.

An alternative process for the synthesis of alcohols is based on the Guerbet reaction which is known for many decades (M. Guerbet, C. R. Hebd. Séances Acad. Sci. 1899, 128, p. 511-513). It is generally accepted that the mechanism leading to Guerbet alcohols comprises the following three steps: (i) dehydrogenation of a primary alcohol to the respective aldehyde; (ii) aldol condensation of two aldehyde molecules to an α,β-unsaturated aldehyde with elimination of water; and (iii) hydrogenation of the unsaturated aldehyde to the dimer alcohol. An alkaline catalyst, e.g. sodium or potassium hydroxide or sodium or potassium alkoxides, is required for the Guerbet reaction. Often homogeneous or hetereogeneous metal catalysts are added to accelerate the dehydrogenation and hydrogenation steps. However, the Guerbet reaction generally suffers from harsh conditions, poor selectivity, separation issues and low yield.

In the chemical industry, 1-butanol is an important intermediate product and solvent for a broad variety of products, including paints and various plastics. Up to now, 1-butanol is produced from a petro-based feedstock, leading to a significant product carbon footprint for 1-butanol and the resulting products. Therefore, it is important for the chemical industry to find and open an economical and sustainable process route to 1-butanol with a lower product carbon footprint.

Ethanol may be a sustainable feedstock to produce chemicals. Using ethanol in the Guerbet reaction may be a profitable and sustainable approach to produce butanol. Whereas the Guerbet reaction is used up to date to produce higher alcohols from higher boiling alcohol feedstocks than ethanol, there is so far no industrial usage for the Guerbet reaction for ethanol as the feedstock to produce 1-butanol. While the Guerbet reaction itself may seem a simple chemical reaction, employing ethanol as the feedstock causes inherent problems particularly concerning selectivity. Because the product, 1-butanol, can itself also undergo dehydrogenation, higher alcohols often result as side products in the process, making the reaction so far not profitable on an industrial scale. A further optimization of said process to turn said process profitable on an industrial scale is thus desired.

In the Guerbet reaction of ethanol to 1-butanol, a reaction mixture is obtained which generally contains the desired product 1-butanol, and further unreacted ethanol, water, remaining base, ester formed during the reaction as a side product, such as an acetate, the homogeneous Guerbet-catalyst, salts formed from the residues of such catalyst, as well as a mixture of higher alcohols. A concept for the work-up of said reaction mixture comprises, as a first step, the removal of any unreacted ethanol and water. From the so obtained mixture containing mainly 1-butanol, remaining base, side products such as the ester, the homogenous Guerbet-catalyst as well as higher alcohols, at least part of the ester and at least part of remaining base are removed by extraction with water in order to avoid their accumulation in the further work-up process. When extracting at least part of the ester and remaining base with water, however, on an industrial scale, a large volume of fresh water is required.

In addition to the environmental strain to provide such large amounts of fresh water, also a large amount of waste water is created. In the waste water, potentially, a high total organic content (TOC) may remain, which needs to be removed to avoid pollution of the environment, further adding to the overall costs of the process. In addition, the TOC, after removal from the waste water, is treated as waste material, which results in a loss of valuable carbon material.

Therefore, it was an object of the present invention to provide a process for producing an acid, and in particular to provide an alcohol conversion process employing a homogeneous catalyst and a base, preferably employing a bio-based alcohol, wherein an ester is formed, wherein an acid from the ester is obtained, and wherein less waste water with a high total organic content is created.

The present invention thus relates to a process for producing an acid, and in particular to an alcohol conversion process employing a homogeneous catalyst and a base, wherein an ester is formed as a byproduct of said alcohol conversion process, and wherein an acid from the ester is obtained. It has been found that a specific separation step of the ester during the work-up advantageously results in gaining valuable carbon material from waste of said alcohol conversion process, said bio-carbon material suitable for various applications, while creating less waste water with a high total organic content. In case bio alcohols, e.g. alcohols obtainable from biological sources, such as sugar or corn, are employed in the alcohol conversion process, valuable bio-carbon material may be gained even further reducing the environmental strain. In a preferred embodiment, the present invention relates to a process for producing acetic acid as a side product from an alcohol conversion process employing a homogeneous catalyst and a base using ethanol as an educt, preferably bio-ethanol.

The present invention in particular relates to a process for producing an acid, preferably acetic acid, more preferably bio acetic acid, comprising
(i) providing a chemical component C comprising one or more of a catalyst, a precursor of the catalyst, a reduced form of the catalyst, and a reduced form of the precursor of the catalyst;
(ii) preparing a liquid mixture M_{E} comprising at least one alcohol R-CH₂-CH₂-OH with R being H or C₁-C₄-alkyl, a base, and the chemical component C provided in (i);
(iii) subjecting the liquid mixture M_{E} prepared in (ii) to alcohol conversion conditions in a reaction space S_{R}, obtaining in said reaction space a reaction mixture M_{G} comprising at least one alcohol R-CH₂-CH₂-(CHR-CH₂)ₓ-OH with x being an integer in the range of from 1 to 4 and further comprising unreacted alcohol R-CH₂-CH₂-OH and at least one ester R-CH₂-C(O)-O-R' with R' being C₁-C₄-alkyl;
(iv) separating at least part of the at least one alcohol R-CH₂-CH₂-OH from the reaction mixture M_{G} obtained in (iii), obtaining a mixture M_{GS} depleted in the at least one alcohol R-CH₂-CH₂-OH and comprising the at least one alcohol R-CH₂-CH₂-(CHR-CH₂)ₓ-OH and at least one ester R-CH₂-C(O)-O-R';
(v) preparing a mixture M_{GB} comprising an aqueous phase P_{A} and an organic phase Po, comprising admixing water with the mixture M_{GS} obtained in (iv), said aqueous phase P_{A} comprising at least part of the at least one ester R-CH₂-C(O)-O-R' and said organic phase P_{O} comprising at least part of the at least one alcohol R-CH₂-CH₂-(CHR-CH₂)ₓ-OH;
(vi) subjecting the mixture M_{GB} prepared in (v) to phase separation conditions, obtaining an aqueous mixture M_{A} comprising at least part of the at least one ester R-CH₂-C(O)-O-R' and a mixture M_{AC} comprising at least part of the at least one alcohol R-CH₂-CH₂-(CHR-CH₂)ₓ-OH;
(vii) adding an acid component A to the aqueous mixture M_{A} obtained in (vi), obtaining an aqueous mixture M_{AA} comprising an acid R-CH₂-C(O)OH derived from the ester R-CH₂-C(O)-O-R';
(viii) separating at least part of the acid R-CH₂-C(O)OH from the mixture M_{AA} obtained in (vii)
wherein
the acid component A comprises an acid selected from then group consisting of sulfuric acid, nitric acid, citric acid, phosphoric acid, boric acid, carbonic acid, and a mixture of two or more thereof;
the base is selected from the group consisting of alkali hydroxides, alkaline earth hydroxides, alkali carbonates, alkali hydrogen carbonates, alkaline earth carbonates, alkaline hydrogen carbonates, alkali alkoxides, alkaline earth alkoxides, alkali metal amides, alkaline earth metal amides, alkali metal diisopropylamides, alkaline earth metal diisopropylamides, secondary amino acids, and a mixture of two or more thereof;
wherein the catalyst comprises a compound of formula (A) 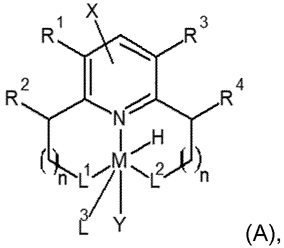 wherein
   M is selected from the group consisting of Ir, Mn, Os, Pd, Pt, Rh, and Ru;
   L¹ and L² are, independently of each other, PR^{a}R^{b}, NR^{a}R^{b}, SR^{a}, SH, S(=O)R^{a}, heteroaryl containing at least one heteroatom selected from nitrogen and sulfur, AsR^{a}R^{b}, SbR^{a}R^{b}, and a N-heterocyclic carbene represented by the structures: 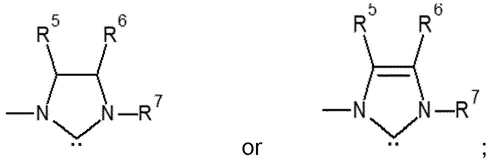
   L³ is selected from the group consisting of CO, PR^{a}R^{b}R^{c}, AsR^{a}R^{b}R^{c}, SbR^{a}R^{b}R^{c}, R^{d}CN, SR^{a}R^{b}, R^{d}NC, N₂, PF₃, pyridine, and thiophene;
   R¹, R², R³ and R⁴ either are hydrogen, or form together with the pyridyl unit of the catalyst of formula (A) an acridinyl unit, or R¹ and R² or R³ and R⁴ form together with the pyridyl unit of the catalyst of formula (A) a quinolinyl unit;
   n is 0 or 1;
   Y is selected from the group consisting of H, F, Cl, Br, I, OC(=O)CF₃, OSO₂CF₃, CN, CO, OH, OR, NR^{d}₂, NH₃, NR^{d}₃, and R^{d}₂NSO₂R^{d};
   R^{a}, R^{b}, R^{c}, R^{d}, R⁵, R⁶ and R⁷ are, independently of each other, selected from the group consisting of H, unsubstituted or substituted C₁-C₁₀-alkyl, wherein the substituents are selected from the group consisting of F, Cl, Br, OH, CN, NH₂, and C₁-C₁₀-alkyl;
   unsubstituted or substituted C₁-C₁₀-cycloalkyl, wherein the substituents are selected from the group consisting of F, Cl, Br, OH, CN, NH₂, and C₁-C₁₀-alkyl; unsubstituted or substituted C₃-C₁₀-heterocyclyl comprising at least one heteroatom selected from the group consisting of N, O, and S, wherein the substituents are selected from the group consisting of F, Cl, Br, OH, CN, NH₂, and C₁-C₁₀-alkyl; unsubstituted or substituted C₅-C₁₀-aryl, wherein the substituents are selected from the group consisting of F, Cl, Br, OH, CN, NH₂, and C₁-C₁₀-alkyl; and unsubstituted or substituted C₅-C₁₀-heteroaryl comprising at least one heteroatom selected from the group consisting of N, O, and S, wherein the substituents are selected from the group consisting of F, Cl, Br, OH, CN, NH₂ and C₁-C₁₀-alkyl; and
   X is selected from the group consisting of one, two, three, four, five, six, and seven substituents positioned at any carbon atom on the acridinyl unit, or one, two, three, four and five substituents positioned at any carbon atom on the quinolinyl unit, or one substituent positioned at the carbon atom on the pyridyl unit, wherein the substituents are selected from the group consisting of F, Cl, Br, OH, CN, NH₂, and C₁-C₁₀-alkyl;
   the precursor of the catalyst comprising a compound of formula (A) comprises a mixture comprising a compound comprising a metal M and at least one component selected from the group consisting of CO, PR^{a}R^{b}R^{c}, SR^{a}R^{b}, R^{a}CN, R^{a}NC, N₂, PF₃, organic carbonyl compounds, C₁-C₁₀-alkyl, C₁-C₁₂-cycloalkyl, C₂-C₁₂-alkenyl, C₃-C₁₅-cycloalkenyl, C₅-C₂₀-aryl, CN, CO, OH, OC(=O)CF₃, OSO₂CF₃, hydrides, pyridines, halogenides, hydroxides, and thiophenes; and a compound of formula (H) 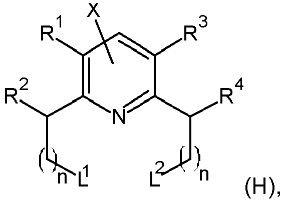
   M is selected from the group consisting of Ir, Mn, Os, Pd, Pt, Rh, and Ru;
   L¹ and L², are, independently of each other, PR^{a}R^{b}, NR^{a}R^{b}, SR^{a}, SH, S(=O)R^{a}, heteroaryl containing at least one heteroatom selected from nitrogen and sulfur, AsR^{a}R^{b}, SbR^{a}R^{b}, and a N-heterocyclic carbene represented by the structures: 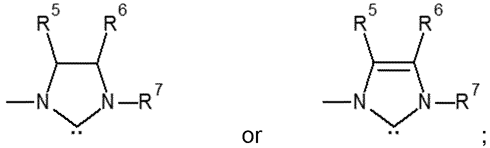
   R¹, R², R³ and R⁴ either are hydrogen, or form together with the pyridyl unit of the catalyst of formula (A) an acridinyl unit, or R¹ and R² or R³ and R⁴ form together with the pyridyl unit of the catalyst of formula (A) a quinolinyl unit;
   n is 0 or 1;
   R^{a}, R^{b}, R^{c}, R^{d}, R⁵, R⁶ and R⁷ are, independently of each other, selected from the group consisting of H, unsubstituted or substituted C₁-C₁₀-alkyl, wherein the substituents are selected from the group consisting of F, Cl, Br, OH, CN, NH₂, and C₁-C₁₀-alkyl;
   unsubstituted or substituted C₁-C₁₀-cycloalkyl, wherein the substituents are selected from the group consisting of F, Cl, Br, OH, CN, NH₂, and C₁-C₁₀-alkyl; unsubstituted or substituted C₃-C₁₀-heterocyclyl comprising at least one heteroatom selected from the group consisting of N, O, and S, wherein the substituents are selected from the group consisting of F, Cl, Br, OH, CN, NH₂, and C₁-C₁₀-alkyl; unsubstituted or substituted C₅-C₁₀-aryl, wherein the substituents are selected from the group consisting of F, Cl, Br, OH, CN, NH₂, and C₁-C₁₀-alkyl; and unsubstituted or substituted C₅-C₁₀-heteroaryl comprising at least one heteroatom selected from the group consisting of N, O, and S, wherein the substituents are selected from the group consisting of F, Cl, Br, OH, CN, NH₂ and C₁-C₁₀-alkyl;
   X is selected from the group consisting of one, two, three, four, five, six, and seven substituents positioned at any carbon atom on the acridinyl unit, or one, two, three, four and five substituents positioned at any carbon atom on the quinolinyl unit, or one substituent positioned at the carbon atom on the pyridyl unit, wherein the substituents are selected from the group consisting of F, Cl, Br, OH, CN, NH₂, and C₁-C₁₀-alkyl.

Preferably, the acid component A comprises an acid selected from the group consisting of sulfuric acid, nitric acid, phosphoric acid, boric acid, and a mixture of two or more thereof. More preferred is that the acid component A comprises nitric acid, phosphoric acid or sulfuric acid, more preferably wherein the acid component A comprises sulfuric acid. Also, the acid component A may comprise, either alternative to the afore-mentioned acids or in combination therewith, hydrochloric acid, hydrobromic acid, or a combination thereof.

In a preferred embodiment, (vii) comprises adding the acid component A to a pH of the aqueous mixture M_{A} in the range of from 1 to 5, more preferably of from 1 to 4, more preferably of from 1 to 3.

It is also preferred that separating at least part of the acid R-CH₂-C(O)OH from the mixture M_{AA} in (viii) comprises stripping, distillation or membrane separation.

Preferably, R is H, integer x is 1 and the acid R-CH₂-C(O)OH is acetic acid.

The amount of the at least one ester R-CH₂-C(O)-O-R' prepared in the reaction mixture M_{G} in (iii) is preferably in a range of from 1 to 10 mol%, more preferably 1 to 8%, and more preferably 2 to 8 mol%, based on 100 mol% of the at least one alcohol R-CH₂-CH₂-OH.

The base is preferably selected from the group consisting of alkali hydroxides, alkali alkoxides, and a mixture thereof. More preferred is the alkali hydroxide being selected from the group consisting of NaOH, KOH, and a mixture thereof, more preferably wherein the alkali hydroxide is KOH. It is also preferred that the alkali alkoxide is selected from the group consisting of sodium alkoxides, potassium alkoxides, and a mixture thereof, preferably from the group consisting of sodium ethoxide, potassium ethoxide, and a mixture thereof.

In a preferred embodiment, integer x is 1 or 2, preferably wherein integer x is 1.

The aqueous phase P_{A} obtained in (v) preferably comprises from 50 to 100 % of the at least one ester R-CH₂-C(O)-O-R', more preferably from 60 to 100 % of the at least one ester R-CH₂-C(O)-O-R', more preferably from 70 to 100 % of the at least one ester R-CH₂-C(O)-O-R', more preferably from 80 to 100 % of the at least one ester R-CH₂-C(O)-O-R', based on 100 % of the ester R-CH₂-C(O)-O-R' content in in the mixture M_{GS}.

In a preferred embodiment, the process further comprises
(ix) recycling at least a part of the separated at least one alcohol R-CH₂-CH₂-OH from the reaction mixture M_{G} obtained in (iv) to the liquid mixture M_{E} in (ii) or in (iii).

It is also preferred that the process further comprises
(x) separating at least part of the at least one alcohol R-CH₂-CH₂-(CHR-CH₂)ₓ-OH from the mixture M_{AC} obtaining a mixture M_{ACS} depleted in the at least one alcohol R-CH₂-CH₂-(CHR-CH₂)ₓ-OH. More preferred is that separating at least part of the at least one alcohol R-CH₂-CH₂-(CHR-CH₂)ₓ-OH in step (x) comprises subjecting the mixture M_{AC} to distillation. The distillation conditions preferably comprise a temperature in the range of from 70 to 180 °C, more preferably from 80 to 160 °C, more preferably from 90 to 150 °C, more preferably from 100 to 140 °C. The distillation conditions also preferably comprise a pressure in the range of from 1 x 10³ to 2 x 10⁵ Pa, preferably from 2 x 10³ to 1.7 x 10⁵ Pa more preferably from 3 x 10³ to 1.4 x 10⁵ Pa, more preferably from 5 x 10³ to 1 x 10⁵ Pa.

The alcohol conversion conditions in (iii) preferably comprise a temperature of the reaction mixture M_{G} in the range of from 100 to 250 °C and a pressure in the reaction space S_{G} in the range of from 1 x 10⁵ to 4 x 10⁶ Pa. It is also preferred that the alcohol conversion conditions according to (iii) comprise a temperature of the reaction mixture M_{G} in the range of from 100 to 200 °C, more preferably in the range of from 120 to 180 °C, more preferably in the range of from 120 to 170 °C, more preferably in the range of from 140 to 170 °C.

The process is preferably a continuous process. Alternatively, the process is preferably a semi-batch process or a batch process.

The alcohol conversion conditions according to (iii) preferably comprise the presence of at least one inert gas in the reaction space S_{R}, wherein the at least one inert gas is preferably selected from the group consisting of nitrogen, argon, and a mixture thereof.

It is also preferred that the alcohol conversion conditions according to (iii) comprise a pressure in the reaction space S_{R} in the range of from 1 x 10⁵ to 3.5 x 10⁶ Pa, more preferably in the range of from 1 x 10⁵ to 3.1 x 10⁶ Pa, more preferably in the range in the range of from 1 x 10⁵ to 2 x 10⁶ Pa, more preferably in the range in the range from 1 x 10⁵to 1.5 x 10⁶ Pa.

In (iii), the reaction space S_{R} preferably comprises a gas phase, wherein said gas phase comprises H₂, more preferably wherein the H₂ partial pressure of the gas phase in the reaction space S_{G} is maintained in the range of from 2 x 10⁴ to 3.1 x 10⁶ Pa, more preferably in the range of from 2 x 10⁴ to 1.1 x 10⁶ Pa, more preferably in the range of from 2 x 10⁴ to 6 x 10⁵ Pa. The H₂ partial pressure of the gas phase is preferably maintained by relaxation of the gas phase or by introducing H₂ into the gas phase. Also, the H₂ partial pressure of the gas phase is preferably maintained by relaxation of the gas phase.

"Maintaining" the H₂ partial pressure of the gas phase in the sense of the present invention includes ensuring that the H₂ partial pressure is within the desired range during the reaction. In case the H₂ partial pressure is within the desired range, no active steps have to be carried out mandatorily, but the pressure may still be adjusted to a different part of the range if desired. However, in order to ensure that the H₂ partial pressure is neither too high nor too low, the H₂ partial pressure may preferably be adjusted, or must be adjusted in case of ensuring that the H₂ partial pressure is maintained within the desired range, for example by relaxation of the gas phase, in which case the H₂ partial pressure may be reduced, or, alternatively, by introducing H₂ into the gas phase, in which case the H₂ partial pressure may be increased. Depending upon the H₂ partial pressure during the reaction, one or even both of said alternatives may be carried out if desired to adjust the H₂ partial pressure and to maintain the H₂ partial pressure within the desired pressure range at all times during the reaction.

The pressure during the reaction can be monitored by, for example, determination of the overall pressure and comparison to the starting pressure. As hydrogen tends to build up during the reaction, the Hz partial pressure changes, e.g. increases, resulting in the pressure to increase over time. For example, by actively measuring and controlling the overall pressure during the reaction, it may be ensured that the H₂ partial pressure is within the claimed range. If the overall pressure built up is too high, this tends to be at least in part the result of the H₂ partial pressure increasing. By relaxation of the gas phase, hydrogen can be removed from the gas phase and the H₂ partial pressure can be maintained in the desired range. Thus, in one preferred embodiment, the H₂ partial pressure of the gas phase is preferably maintained in the respective range by monitoring the overall pressure of the reaction and adjusting the overall pressure if required, preferably by relaxation of the gas phase, in which case the H₂ partial pressure may be reduced, or, alternatively, by introducing H₂ into the gas phase, in which case the H₂ partial pressure may be increased.

Alternatively, the hydrogen partial pressure can be determined by other means, such as taking samples of the gas phase during the reaction and analyzing same. As another alternative, the pressure may be monitored via online measurement, and adjusted accordingly as outlined above.

Preferably, the liquid mixture M_{E} prepared according to (ii) further comprises a solvent component S. The solvent component S preferably comprises a solvent which has a boiling point of 110 °C or more, more preferably a boiling point of 140 °C or more, more preferably a boiling point of 160 °C or more, more preferably a boiling point of 180 °C or more, more preferably a boiling point of 190 °C or more.

The solvent in the solvent component S preferably has a solubility in water at 25 °C of from 0 to 0.7 weight-%, more preferably a solubility in water at 25 °C of from 0 to 0.5 weight-%, more preferably a solubility in water at 25 °C of from 0 to 0.1 weight-%, more preferably a solubility in water at 25 °C of from 0 to 0.05 weight-%.

In another preferred embodiment, a distribution coefficient of the catalyst in a system of the solvent component S and water is from 0 to 0.01, more preferably from 0 to 0.005, more preferably from 0 to 0.005, based on 1 kg catalyst.

The solvent component S preferably comprises a mixture of at least two solvents with a boiling point of 140 °C or more, more preferably with a boiling point of 160 °C or more, more preferably with a boiling point of 180 °C or more, more preferably with a boiling point of 190 °C or more. In a further preferred embodiment, the solvent component S comprises a solvent which is selected from the group consisting of biphenyl, diphenyl ether, 1-tert-butyl-3,5-dimethyl-benzene, ethylbenzene, cyclododecane, cyclononane, cyclooctane, cycloheptane, decaline, n-butylbutyrate, n-hexylhexyrate, n-octyloctyrate, texanole, di-n-butylether, di-iso-butylether, di-sec-butylether, 1-hexanol, 1-octanol, 1-decanol, 1-dodedacanol, 2-ethylbutan-1-ol, 2-ethylhexan-1-ol, 2-ethyloctan-1-ol, 2-ethyldecan-1-ol, 2-ethyldodecan-1-ol, 2-butylhexan-1-ol, 2-butyloctan-1-ol, 2-butyldecan-1-ol, 2-butyldodecan-1-ol, 2-hexyldecanol, 2-octyldodecanol, 2-propylheptan-1-ol, and a mixture of two or more thereof; preferably wherein the solvent component S comprises at least one solvent selected from the group consisting of 2-ethylbutan-1-ol, 2-ethylhexan-1-ol, 2-ethyloctan-1-ol, 2-ethyldecan-1-ol, 2-ethyldodecan-1-ol, 2-butylhexan-1-ol, 2-butyloctan-1-ol, 2-butyldecan-1-ol, 2-butyldodecan-1-ol, 2-hexadecanol, 2-octyldodecanol, 2-propylheptan-1-ol, and a mixture of two or more thereof.

Preferably, the solvent does not form an azeotrope with water. An azeotrope or a constant heating point mixture is a mixture of two or more components in fluidic states whose proportions cannot be altered or changed by simple distillation. This happens because when an azeotrope is boiled, the vapor has the same proportions of constituents as the unboiled mixture. Each azeotrope has a characteristic boiling point. It is not possible to separate the components by fractional distillation.

Preferably, the solvent component S does not include any one of benzene, toluene, xylene or mesitylene.

It is preferred that the alcohol conversion conditions according to (iii) comprise an amount of the solvent component S in the reaction mixture M_{G} in the range of from 5 to 50 weight-%, more preferably in the range of from 5 to 30 weight-%, more preferably in the range of from 5 to 10 weight-%, based on the total weight of the reaction mixture M_{G}.

It is furthermore preferred that from 90 to 100 weight-%, more preferably from 95 to 100 weight-%, more preferably from 98 to 100 weight-%, more preferably from 99 to 100 weight-% of the liquid mixture M_{E} prepared according to (ii) consist of the at least one alcohol R-CH₂-CH₂-OH, the base, the solvent component S and the chemical component C.

Preferably, the mixture M_{ACS} obtained according to (x) further comprises at least part of the chemical component C, and preferably further comprises at least part of the solvent component S.

The process preferably further comprises
(xi) recycling at least a part of the solvent component S comprised in the mixture M_{ACS} obtained according to (x) to (ii) or (iii).

Even further preferred is that the process comprises
(xii) recycling at least a part of the solvent component S and at least a part of the chemical component C comprised in the mixture M_{ACS} obtained according to (x) to (ii) or (iii).

In formula (A), n is preferably 0 if R¹, R², R³ and R⁴ are hydrogen.

In another preferred embodiment, the reaction mixture M_{G} in (iii) further comprises water; more preferably wherein the amount of water in reaction mixture M_{G} is 0.2 weight-% or less, more preferably in the range of from 0 to 0.2 weight-%, more preferably from 0.0001 to 0.2 weight-%, more preferably from 0.0001 to 0.15 weight-%, more preferably from 0.0005 to 0.1 weight-%, more preferably from 0.0005 to 0.08 weight-%, more preferably from 0.0005 to 0.05 weight-%, based on the total weight-% of the reaction mixture M_{G}. It is even more preferred that step (iii) further comprises at least partially removing of water from the reaction mixture reaction mixture M_{G}, more preferably the continuous removal of at least a part of water from the reaction mixture reaction mixture M_{G}.

Preferably, the chemical component C comprises a compound of formula (B) 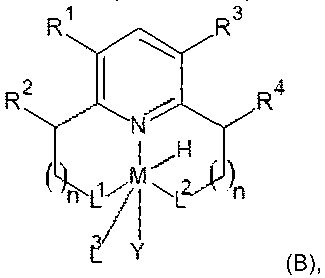 wherein
M is selected from the group consisting of Ir, Mn, Os, Pd, Pt, Rh, and Ru;
L¹ and L² are, independently of each other, PR^{a}R^{b}, NR^{a}R^{b}, SR^{a}, SH, and S(=O)R^{a};
L³ is selected from the group consisting of CO, PR^{a}R^{b}R^{c}, SR^{a}R^{b}, R^{a}CN, R^{a}NC, N₂, PF₃, pyridine, and thiophene;
R¹, R², R³ and R⁴ either are hydrogen, or form together with the pyridyl unit of the catalyst of formula (A) an acridinyl unit;
n is 0 or 1, and if R¹, R², R³ and R⁴ are hydrogen, n is 0;
R^{a}, R^{b}, R^{c} and R^{d} are, independently of each other, selected from the group consisting of H, unsubstituted or substituted C₁-C₁₀-alkyl wherein the substituents are selected from the group consisting of F, Cl, Br, OH, CN, NH₂, and C₁-C₁₀-alkyl; unsubstituted or substituted C₁-C₁₀-cycloalkyl wherein the substituents are selected from the group consisting of F, Cl, Br, OH, CN, NH₂, and C₁-C₁₀-alkyl; C₃-C₁₀-heterocyclyl comprising at least one heteroatom selected from the group consisting of N, O, and S; C₅-C₁₀-aryl; and C₅-C₁₀-heteroaryl comprising at least one heteroatom selected from the group consisting of N, O, and S;
Y is selected from the group consisting of H, F, Cl, Br, I, OC(=O)CF₃, OSO₂CF₃, CN, CO, and OH;
and wherein for the compound of formula (L), R¹, R², R³ and R^{4,} L¹, L² and n are preferably identical to R¹, R², R³ and R⁴, L¹, L² and n of the catalyst of formula (B).

Also preferred is that the chemical component C comprises a compound of formula (C) 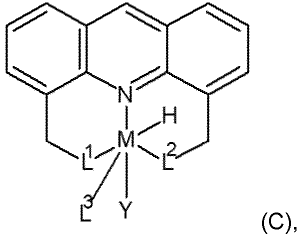 wherein
M is selected from the group consisting of Ir, Ru, and Mn;
L¹ and L² are, independently of each other, PR^{a}R^{b}, NR^{a}R^{b}, SR^{a}, SH, and S(=O)R^{a};
L³ is selected from the group consisting of CO, PR^{a}R^{b}R^{c}, SR^{a}R^{b}, R^{a}CN, R^{a}NC, N₂, PF₃, pyridine, and thiophene;
R^{a}, R^{b}, R^{c} and R^{d} are, independently of each other, selected from the group consisting of H, unsubstituted or substituted C₁-C₁₀ alkyl wherein the substituents are selected from the group consisting of F, Cl, Br, OH, CN, NH₂, and C₁-C₁₀ alkyl; unsubstituted or substituted C₁-C₁₀-cycloalkyl wherein the substituents are selected from the group consisting of F, Cl, Br, OH, CN, NH₂, and C₁-C₁₀ alkyl; C₃-C₁₀ heterocyclyl comprising at least one heteroatom selected from the group consisting of N, O, and S; C₅-C₁₀ aryl; and C₅-C₁₀ heteroaryl comprising at least one heteroatom selected from the group consisting of N, O, and S;
Y is selected from the group consisting of H, F, Cl, Br, I, OC(=O)CF₃, OSO₂CF₃, CN, CO, and OH;
and wherein for the compound of formula (L), R¹, R², R³ and R⁴, L¹, L², and n are preferably identical to R¹, R², R³ and R⁴, L¹, L², and n of the catalyst of formula (C).

It is furthermore preferred that the chemical component C comprises a compound of formula (D) 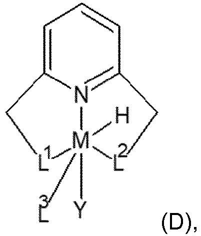 wherein
M is selected from the group consisting of Ir, Ru, and Mn;
L¹ and L² are, independently of each other, PR^{a}R^{b}, NR^{a}R^{b}, SR^{a}, SH, and S(=O)R^{a};
L³ is selected from the group consisting of CO, PR^{a}R^{b}R^{c}, SR^{a}R^{b}, R^{a}CN, R^{a}NC, N₂, PF₃, pyridine, and thiophene;
R^{a}, R^{b}, R^{c} and R^{d} are, independently of each other, selected from the group consisting of H, unsubstituted or substituted C₁-C₁₀-alkyl wherein the substituents are selected from the group consisting of F, Cl, Br, OH, CN, NH₂, and C₁-C₁₀-alkyl; unsubstituted or substituted C₁-C₁₀-cycloalkyl wherein the substituents are selected from the group consisting of F, Cl, Br, OH, CN, NH₂, and C₁-C₁₀-alkyl; C₃-C₁₀-heterocyclyl comprising at least one heteroatom selected from the group consisting of N, O, and S; C₅-C₁₀ aryl; and C₅-C₁₀-heteroaryl comprising at least one heteroatom selected from the group consisting of N, O, and S;
Y is selected from the group consisting of H, F, Cl, Br, I, OC(=O)CF₃, OSO₂CF₃, CN, CO, and OH;
and wherein for the compound of formula (L), R¹, R², R³ and R⁴, L¹, L² and n are preferably identical to R¹, R², R³ and R⁴, L¹, L² and n of the catalyst of formula (D).

Moreover, it is preferred that M is selected from the group consisting of Ir and Ru, more preferably wherein M is Ru.

Preferably, L³ is CO.

Preferably, L¹ and L² are each (PR^{a}R^{b}), and wherein R^{a} and R^{b} are C₁-C₁₀-alkyl, more preferably wherein R^{a} and R^{b} are each isopropyl or tert-butyl. Alternatively, it is preferred that L¹ and L² are each (PR^{a}R^{b}), and wherein R^{a} and R^{b} are C₁-C₁₀-cycloalkyl, more preferably wherein R^{a} and R^{b} are each cyclohexyl. Also, alternatively, it is preferred that L¹ and L² are each (PR^{a}R^{b}), and wherein R^{a} and R^{b} are C₅-C₁₀-aryl.

Preferably, Y is selected from the group consisting of F, Cl, Br and I, more preferably wherein Y is selected from the group consisting of Cl or Br, more preferably wherein Y is CI. It is also preferred that Y is CO.

In yet another preferred embodiment, the chemical component C comprises a compound of formula (E) 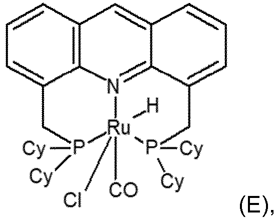 wherein Cy is cyclohexyl.

Preferably, the reduced form of the catalyst comprises a compound of formula (E') 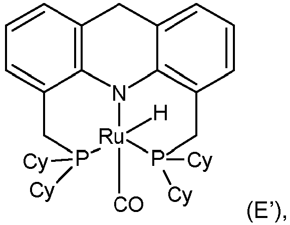 wherein Cy is cyclohexyl.

It is also preferred that the chemical component C comprises a compound of formula (F) 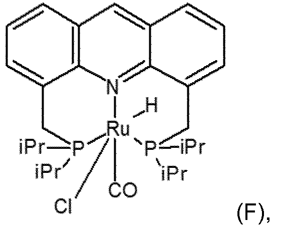 wherein iPr is isopropyl.

It is moreover preferred that the reduced form of the catalyst comprises a compound of formula (F') 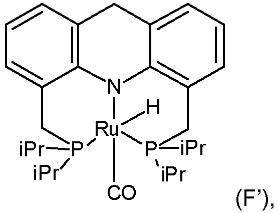 wherein iPr is isopropyl.

It is also preferred that the chemical component C comprises a compound of formula (G) 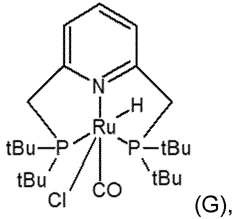 wherein tBu is tert-butyl.

The reduced form of the catalyst preferably comprises a compound of formula (G') 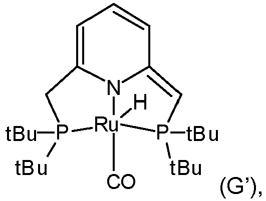 wherein tBu is tert-butyl.

The chemical component C preferably comprises a compound comprising a metal M selected from the group consisting of IrCl₃ x HzO, [Ir(COD)Cl]₂, [Ir(COE)zCl]z, [Ir(C₂H₄)₂Cl]₂, [Ir(COD)OH]₂, [Ir(COD)MeO]₂, [IrCp*Cl₂], [IrCp Cl₂], Ir₄(CO)₁₂, [Ir(PPh₃)₂(CO)Cl], [Ir(acetylacetonate)₃], and [lr(acetylacetonate)(COD)], wherein Cp is cylclopentadienyl, Cp* is pentamethylcyclopentadienyl, COD is 1,5-cyclooctadienyl, COE is cyclooctenyl, and methylallyl is 2-methylallyl. It is also preferred that the chemical component C comprises a compound comprising a metal M selected from the group consisting of [Ru(p-cymene)Cl₂]₂, [Ru(benzene)Cl₂]_{y}, [Ru(CO)₂Cl₂]_{y}, where y is in each case in the range from 1 to 1000, [Ru(CO)₃Cl₂]₂, [Ru(COD)(allyl)₂], RuCl₃ x H₂O, [Ru(acetylacetonate)₃], [Ru(DMSO)₄Cl₂], [Ru(cyclopentadienyl)(CO)zCl], [Ru(cyclopentadienyl)(CO)₂H], [Ru(cyclopentadienyl)(CO)₂]₂, [Ru(Cp)(CO)₂Cl], [Ru(Cp*)(CO)₂H], [Ru(Cp*)(CO)₂]₂, [Ru(indenyl)(CO)₂Cl], [Ru(indenyl)(CO)₂H], [Ru(indenyl)(CO)₂]₂, ruthenocene, [Ru(COD)Cl₂]₂, [Ru(Cp*)(COD)CI], [Ru₃(CO)₁₂], [Ru(PPh₃)₄(H)₂], [Ru(PPh₃)₃(Cl)₂], [Ru(PPh₃)₃(CO)(Cl)₂], [Ru(PPh₃)₃(CO)(Cl)(H)], [Ru(PPh₃)₃(CO)(H)₂], and [Ru(cyclooctadienyl)(methylallyl)₂], wherein Cp is cylclopentadienyl, Cp* is pentamethylcyclopentadienyl, COD is 1,5-cyclooctadienyl, and methylallyl is 2-methylallyl.

In yet another preferred embodiment, the reduced form of the precursor comprises a compound of formula (P-I) or (P-II): 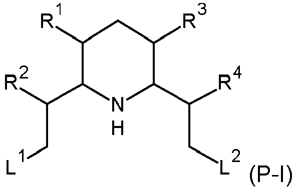
wherein R¹, R², R³ and R⁴ either are hydrogen, or form together with the N-containing ring a tetrahydroquinoline unit, a decahydroquinoline unit, a tetrahydroacridine unit, or a tetradecahydroacridine unit; and
wherein L¹ and L² are, independently of each other, as defined above; 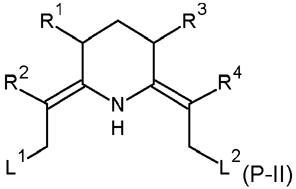
wherein R¹, R², R³ and R⁴ are hydrogen; and
wherein L¹ and L² are, independently of each other, as defined above.

It is also preferred that the reduced form of the precursor comprises a compound of formula (P-I): 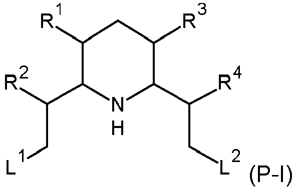 wherein R¹, R², R³ and R⁴ either are hydrogen, or form together with the N-containing ring a tetrahydroacridine unit, or a tetradecahydroacridine unit.

It is furthermore preferred that the reduced form of the precursor comprises a compound of formula (P-II): 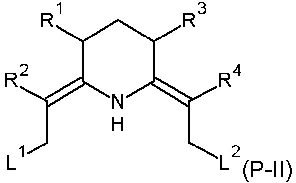
wherein R¹, R², R³ and R⁴ are hydrogen; and
wherein L¹ and L² are, independently of each other, as defined above.
R is preferably selected from the group consisting of H, methyl, ethyl, propyl, isopropyl, butyl, isobutyl, and tert-butyl, more preferably from the group consisting of H, methyl, ethyl, propyl, and isopropyl, more preferably from the group consisting of H, ethyl, and propyl, wherein even more preferably R is H.

The liquid mixture M_{E} prepared according to (ii) preferably further comprises a compound of formula (H): 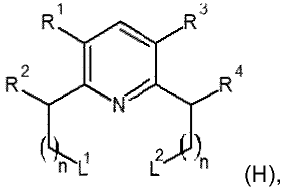 wherein R¹, R², R³ and R⁴, L¹, L², and n are identical to R¹, R², R³ and R⁴, L¹, L², and n of the catalyst of formula (A). More preferably, in the liquid mixture M_{E} prepared according to (ii) and subjected to alcohol version conditions according to (iii), the molar ratio of the compound of formula (H) relative to the compound of formula (A) is in a range of from 0.01:1 to 10:1, more preferably in the range of from 0.05:1 to 10:1, more preferably in the range of from 0.1:1 to 10:1, more preferably in the range of from 0.1:1 to 10:1, more preferably in the range of from 0.3:1 to 10:1, more preferably in the range of from 0.5:1 to 10:1, more preferably in the range of from 0.7:1 to 10:1, more preferably in the range of from 0.8:1 to 10:1, more preferably in the range of from 1:1 to 10:1 more preferably in the range of from 1.01:1 to 10:1, more preferably in the range of from 1.02:1 to 8:1, more preferably in the range from 1.03:1 to 7:1, more preferably in the range from 1.04:1 to 6:1, and more preferably in the range from 1.05:1 to 5:1.

Preferably, the compound of formula (H) is selected from the group consisting of dicyclohexyl-[[5-(dicyclohexylphosphanylmethyl)acridin-4-yl]methyl]phosphane, diisopropyl-[[5-(diisopropylphosphanylmethyl)acridin-4-yl]methyl]phosphane, dicyclohexyl-[[5-(dicyclohexylphosphanylmethyl)pyridin-4-yl]methyl]phosphane and diisopropyl-[[5-(diisopropylphosphanylmethyl)pyridin-4-yl]methyl]phosphane, more preferably the compound of formula (H) is cyclohexyl-[[5-(dicyclohexylphosphanylmethyl)acridin-4-yl]methyl]phosphane or diisopropyl-[[5-(diisopropylphosphanylmethyl)acridin-4-yl]methyl]phosphane.

The at least one alcohol, preferably the at least one alcohol R-CH₂-CH₂-OH, is preferably a bio-based alcohol, more preferably obtainable or obtained from sugar-containing crops, more preferably from one or more of sugar cane and corn. It is also preferred that the at least one alcohol R-CH₂-CH₂-OH is a bio-alcohol obtained by fermentation.

In the process in accordance with the present invention, the reaction space S_{R} is comprised in a reactor vessel, wherein the reactor vessel is preferably a complete-mixing reactor vessel.

According to a further aspect, the present invention relates to a process, preferably to the process as described above, which comprises the step of converting a chemical material obtainable by or obtained by the process as described herein to obtain a product product Ω.

Preferably, the product Ω is selected from:
- building block or monomer; or
- polymer, preferably polymer A, polymer composition, preferably polymer composition A, or polymer product, preferably polymer product A; or
- industrial use polymer, industrial use surfactant, descaling compound, industrial use biocide, industrial use solvent, industrial use dispersant, composition thereof or formulation thereof; or
- agrochemical composition, agrochemical formulation auxiliary or agrochemically active ingredient; or
- active pharmaceutical ingredient or intermediate thereof, pharmaceutical excipient, animal feed additive, human food additive, dietary supplements, aroma chemical or aroma composition; or
- aqueous polymer dispersion, preferably polyurethane or polyurethane - poly(meth)acrylate hybrid polymer dispersion, emulsion, binder for paper and fiber coatings, UV-curable acrylic polymer for hot melts and coatings polyisocyanates, hyperbranched polyester polyol, polymeric dispersant for inorganic binder compositions, unsaturated polyester polyol or 100% curable composition; or
- cosmetic surfactant, emollient, wax, cosmetic polymer, UV filter, further cosmetic ingredient or composition or formulation thereof; or
- polymer B, polymer composition B, coating composition, other functional composition, foil, molded body, coating or coated substrate.

Regarding this process from which the product Ω, is obtained, it is preferred:
that the content of the chemical material in the product Ω is 1 weight-% or more, preferably 2 weight-% or more, more preferably 5 weight-% or more, more preferably 15 weight-% or more, more preferably 30 weight-% or more, more preferably 40 weight-% or more, more preferably 60 weight-% or more, more preferably 80 weight-% or more, more preferably 90 weight-% or more, more preferably 95 weight-% or more; and/or
that the content of the chemical material in the product Ω is 100 weight-% or less, preferably 95 weight-% or less, more preferably 90 weight-% or less, more preferably 50 weight-% or less, more preferably 25 weight-% or less, more preferably 10 weight-% or less; and preferably wherein the content is determined based on identity preservation and/or segregation and/or mass balance and/or book and claim chain of custody models, preferably based on mass balance, preferably the International Sustainability and Carbon Certification (ISCC) standard.

The publication Prior Art Disclosure; Issue 684; paragraphs [1000] to [8005]; ISSN: 2198-4786; published: February 12, 2024 will be regarded as Reference RF1, which is incorporated herein by reference in its entirety. Preferably, the product Ω is a product as described in Reference RF1; paragraphs [1000] to [8005]. Preferably, the process described herein is further a process for the production of a product, preferably product Ω.

The converting step to obtain the product Ω preferably comprises one or more step(s) as described below and can be performed by conventional methods well known to a person skilled in the art. The converting step preferably comprises one or more step(s) selected from:
recycling, preferably depolymerizing, gasifying, pyrolyzing, and/or steam cracking; and/or purifying, preferably crystallizing, (solvent) extracting, distilling, evaporating, hydrotreating, absorbing, adsorbing and/or subjecting to ion exchanger; and/or
assembling, preferably foaming, synthesizing, chemical conversion, chemically transforming, polymerizing and/or compounding; and/or
forming, preferably foaming, extruding and/or molding; and/or
finishing, preferably coating and/or smoothing.

In addition, the one or more step(s) are described in detail in Reference RF1; paragraphs [1000] to [8005].

The term "building block", as used in the context of the product Ω herein, comprises compounds, which are in a gaseous or liquid state under standard conditions of 0 °C and 0.1 MPa. Building blocks are typically used in chemical industry to form secondary products, which provide a higher structural complexity and/or higher molecular weight than the building block on which the secondary product is based. The building block is preferably selected from the group consisting of hydrogen, carbon monoxide, carbon dioxid, ethylene oxide, ethylene glycols, syngas comprising a mixture of hydrogen and carbon monoxide, alkanes, alkenes, alkynes and aromatic compounds. The alkanes, alkenes, alkynes and aromatic compounds comprise in particular 1 to 12 carbon atoms, respectively.

The term "monomer", as used in the context of the product Ω herein, comprises molecules, which can react with each other to form polymer chains by polymerization. The monomer is preferably selected from the group consisting of (meth)acrylic acid, salts of (meth)acrylic acid; in particular sodium, potassium and zinc salts; (meth)acrolein and (meth)acrylates. (Meth)acrylates comprising 1 to 22 carbon atoms are preferred, in particular comprising 1 to 8 carbon atoms. The terms (meth)acrylic acid, (meth)acrolein or (meth)acrylate relate to acrylic acid, acrolein or acrylate and also to methacrylic acid, methacrolein or methacrylate, where applicable. Further, the monomer can be selected from hexamethylenediamine (HMD) and adipic acid.

The building block can further be an intermediate compound. The term "intermediate compound", as used in the context of the product Ω herein, comprises organic reagents, which are applied for formation of compounds with higher molecular complexity. The intermediate compound can be selected for example from the group consisting of phosgene, polyisocyanates and propylene oxide. The polyisocyanates are in particular aromatic di- and polyisocyanates, preferably toluene diisocyanate (TDI) and/or diphenylmethane diisocyanate (MDI).

The building block and the monomer and typical converting step(s) to obtain the building block or monomer are described in more detail in paragraphs [1000] to [1012] of Reference RF1.

The term "polymer A", as used in the context of the product Ω herein, comprises thermoplastic, e.g., polyamide or thermoplastic polyurethane, thermoset, e.g., polyurethane, elastomer, e.g., polybutadiene, or a copolymer or a mixture thereof and is defined in more detail in paragraphs [2001] to [2007] of Reference RF1. The term "polymer composition A", as used in the context of the product Ω herein, comprises all compositions comprising a polymer as described above and one or more additive(s), e.g. reinforcement, colorant, modifier and/or flame retardant, and is defined in more detail in paragraph [2008] of Reference RF1. The term "polymer product A", as used in the context of the product Ω herein, comprises any product comprising the polymer A and/or polymer composition A as described above and is defined in more detail in paragraphs [2009] and [2010] of Reference RF1. The step(s) to obtain the polymer, preferably polymer A, polymer composition, preferably polymer composition A or polymer product, preferably polymer product A is/are described in more detail in paragraph [2011] of Reference RF1.

The term "industrial use polymer", as used in the context of the product Ω herein, comprises rheology, polycarboxylate, alkoxylated polyalkylenamine, alkoxylated polyalkylenimine, polyether-based, dye inhibition and soil release cleaning polymers defined in more detail in paragraphs [3035] to [3044] of Reference RF1. The term "industrial use surfactant", as used in the context of the product Ω herein, comprises non-ionic, anionic and amphoteric industrial use surfactants defined in more detail in paragraphs [3008] to [3034] of Reference RF1. The term "industrial use descaling compound", as used in the context of the product Ω herein, comprises non-phosphate based builders (NPB) and phosphonates (CoP) described in more detail in paragraphs [3001] to [3005] of Reference RF1. The term "industrial use biocide", as used in the context of the product Ω herein, refers to a chemical compound that kills microorganisms or inhibits their growth or reproduction defined in more detail in paragraphs [3006] to [3007] of Reference RF1. The term "industrial use solvent", as used in the context of the product Ω herein, comprises alkyl amides, alkyl lactamides, alkyl esters, lactate esters, alkyl diester, cyclic alkyl diester, cyclic carbonates, aromatic aldehydes and aromatic esters defined in more detail in paragraphs [3045] to [3055] of Reference RF1. The term "industrial use dispersant", as used in the context of the product Ω herein, comprises anionic and non-ionic industrial use dispersants defined in more detail in paragraphs [3056] to [3058] of Reference RF1. The term "composition and/or formulation thereof' with reference to the industrial use polymers, industrial use surfactants, descaling compounds and/or industrial use biocides refers to industrial use compositions and/or institutional use products and/or fabric and home care products and/or personal care products defined in more detail in paragraph [3059] of Reference RF1. The converting step(s) to obtain the industrial use polymer, industrial use surfactant, descaling compound and/or industrial use biocide are defined in more detail in paragraph [3060] of Reference RF1. The converting steps to obtain the industrial use composition or formulation of the industrial use polymer, industrial use surfactant, descaling compound and/or industrial use biocide are defined in more detail in paragraph [3061] of Reference RF1.

The term "agrochemical composition", as used in the context of the product Ω herein, typically relates to a composition comprising an agrochemically active ingredient and at least one agrochemical formulation auxiliary. Examples of agrochemical compositions, active ingredients and auxiliaries are described in more detail in Reference RF1, paragraph [4001]. The agrochemical composition may take the form of any customary formulation. The agrochemical compositions are prepared in a known manner, e.g. described by Mollet and Grubemann, Formulation technology, Wiley VCH, Weinheim, 2001; or Knowles, New developments in crop protection product formulation, Agrow Reports DS243, T&F Informa, London, 2005. The converting step(s) to obtain the agrochemically active ingredients and auxiliaries may be conducted in analogy to the production step(s) of their analogues that are based on petrochemicals or other precursors that are not gained by recycling processes. In addition, conversion to compounds mentioned in sections "Polymer" and "Cosmetic surfactant, emollient, wax, cosmetic polymer, UV filter, further cosmetic ingredient or compositions or formulations thereof' may be performed as described in these sections as well as the respective paragraphs in Reference RF1.

The term active pharmaceutical ingredients and/or intermediates thereof, as used in the context of the product Ω herein, comprises substances that provide pharmacological activity or other direct effect in the diagnosis, cure, mitigation, treatment, or prevention of disease, or to affect the structure or any function of the body. Intermediates thereof are isolated products that are generated during a multi-step route of synthesis of an active pharmaceutical ingredient. The term pharmaceutical excipients, as used in the context of the product Ω herein, comprises compounds or compound mixtures used in compositions for various pharmaceutical applications, which are not substantially pharmaceutically active on itself. Active pharmaceutical ingredients and/or intermediates thereof and pharmaceutical excipients are defined in more detail in paragraph [5001] of Reference RF1. The converting step(s) to obtain the active pharmaceutical ingredients and/or intermediates thereof and pharmaceutical excipients may comprise one or more synthesis steps and can be performed by conventional synthesis and techniques well known to a person skilled in the art.

The terms animal feed additives, human food additives, dietary supplements, as used in the context of the product Ω herein, comprises Vitamins, Pro-Vitamins and active metabolites thereof including intermediates and precursors, especially Vitamin A, B, E, D, K and esters thereof, like acetate, propionate, palmitate esters or alcohols thereof like retinol or salts thereof and any combinations thereof; Tetraterpenes, especially isoprenoids like carotenoids and xanthophylls including their intermediates and precursors as well as mixtures and derivates thereof, especially beta carotene, Canthaxanthin, Citranaxanthin, Astaxanthin, Zeaxanthin, Lutein, Lycopene, Apo-carotenoids, and any combinations thereof; organic acids, especially formic acid, propionic acid and salts thereof, such as sodium, calcium or ammonium salts, and any combinations thereof, such as but not limited to mixtures of formic acid and sodium formiate, propionic acid and ammonium propionate, formic acid and propionic acid, formic acid and sodium formiate and propionic acid, propionic acid and sodium propionate and formic acid and sodium formiate; glycerides of carboxylic acids and short and medium chain fatty acids, conjugated linoleic acids, such as omega-6 fatty acid (C18:2) methyl ester and 1,2-propandiol and beverage stabilizers, such as polyvinylpyrrolidone-polymer or polyvinylimidazole/polyvinylpyrrolidone-copolymer. Animal feed additives, human food additives and dietary supplements are defined in more detail in paragraph [5002] of Reference RF1. The converting step(s) to obtain the animal feed additives, human food additives, dietary supplements may comprise one or more synthesis steps and can be performed by conventional synthesis and techniques well known to a person skilled in the art.

The terms aroma chemical and aroma composition as used in the context of the product Ω herein, comprise a volatile organic substance with a molecular weight between 70-250 g/mol comprising a functional group with a carbon skeleton of C₅-C₁₆ carbon atoms comprising linear, branched, cyclic, for example with a ring size of C₅-C₁₈, bicyclic or tricyclic aliphatic chains and but not necessarily one or more unsaturated structural elements like double bonds, triple bonds, aromatics or heteroaromatics and preferably the one or more additional functional groups are selected from alcohol, ether, ester, ketone, aldehyde, acetal, carboxylic acid, nitrile, thiol, amine. In one aspect, the aroma chemical is a terpene-based aroma chemical, for example selected from monoterpenes and monoterpenoids, sesquiterpenes and sesquiterpenoids, diterpenes, triterpenes or tetraterpenes. Aroma chemicals can be combined with further aroma chemicals to give an aroma composition. Aroma chemicals and aroma compositions are defined in more detail in paragraph [5003] of Reference RF1. The converting step(s) to obtain the aroma chemical and aroma composition may comprise one or more synthesis steps and can be performed by conventional synthesis and techniques well known to a person skilled in the art.

The term "aqueous polymer dispersion", as used in the context of the product Ω herein, comprises aqueous composition(s) comprising dispersed polymer(s) and is defined in more detail in the section [6001] entitled "aqueous polymer dispersion" of Reference RF1. The dispersed polymer(s) may be selected from acrylic emulsion polymer(s), styrene acrylic emulsion polymer(s), styrene butadiene dispersion(s), aqueous dispersion(s) comprising composite particles, acrylate alkyd hybrid dispersion(s), polyurethane(s) (including UV-curable polyurethanes) and polyurethane - poly(meth)acrylate hybrid polymer(s). The term "emulsion polymer", as used in the context of the product Ω herein, comprises polymer(s) made by free-radical emulsion polymerization. Aqueous polyurethane dispersion(s) are defined in more detail in the section [6002] entitled "Polyurethane dispersions" of Reference RF1. UV-curable polyurethane(s) is/are defined in more detail in the section [6017] of Reference RF1. Polyurethane - poly(meth)acrylate hybrid polymer(s) is/are defined in more detail in the section [6016] of Reference RF1.

The term "polymeric dispersant", as used in the context of the product Ω herein, comprises preferably polymer(s) comprising polyether side chain, in particular polycarboxylate ether polymer(s) and polycondensation product(s) defined in more detail in paragraph [6020] entitled "Polymeric dispersant" of Reference RF1.

The converting (polymerization) step(s) to obtain the aqueous polymer dispersion(s) comprising emulsion polymer(s) is/are defined in more detail in the section [6003] entitled "Emulsion polymerization" of Reference RF1.

The converting (polymerization) step(s) to obtain the aqueous polyurethane dispersion(s) is/are defined in more detail in the section [6014] entitled "Process for the preparation of aqueous polyurethane dispersions" and section [6017)] entitled "Aqueous UV-curable polyurethane dispersions, their preparation and use and compositions containing them" of Reference RF1. Composition(s) and uses of aqueous polymer dispersion(s) and of polymeric dispersant(s) are defined in more detail in the following sections of Reference RF1:
section [6004] entitled "Uses of aqueous polymer dispersions",
section [6005] entitled "Binders for architectural and construction coatings"
section [6006] entitled "Binders for paper coating"
section [6007] entitled "Binders for fiber bonding"
section [6008] entitled "Adhesive polymers and adhesive compositions"
section [6015] entitled "Aqueous polyurethane dispersions suitable for use in coating compositions"
section [6016] entitled "Aqueous polyurethane - poly(meth)acrylate hybride polymer dispersions suitable for use in coating compositions"
section [6017] entitled "Aqueous UV-curable polyurethane dispersions, their preparation and use and compositions containing them"
section [6018] entitled "Inorganic binder compositions comprising polymeric dispersants and their use" [6019] 100% curable coating compositions

UV-crosslinkable poly(meth)acrylate(s) and its/their uses are defined in more detail in section [6009] entitled "UV-crosslinkable poly(meth)acrylates for use in UV-curable solvent-free hotmelt adhesives and their use for making pressure-sensitive self-adhesive articles" of Reference RF1.

Polyisocyanate(s), composition(s) comprising them and their uses are defined in more detail in section [6010] entitled "Polyisocyanates" of Reference RF1.

Hyperbranched polyester polyol(s) and its/their uses are defined in more detail in section [6011] entitled "Organic solvent based hyperbranched polyester polyols suitable for use in coating compositions" of Reference RF1. The converting step(s) to obtain the hyperbranched polyester polyols is/are defined in more detail in the section [6012] entitled "Preparation of organic solvent based hyperbranched polyester polyols" of Reference RF1. Coating composition(s) comprising hyperbranched polyester polyol(s), polyisocyanate(s) and additive(s) and substrate(s) coated therewith are defined in more detail in section [6013] entitled "Organic solvent based two component coating compositions comprising hyperbranched polyester polyols and polyisocyanates" of Reference RF1.

Unsaturated polyester polyol(s), solvent-based coating composition(s) comprising said unsaturated polyester polyol(s) and substrate(s) for coating with said coating composition(s) are defined in more detail in section [6018] entitled "Organic solvent based coating composition comprising unsaturated polyester polyols" of Reference RF1.

100% curable coating composition(s) is/are defined in more detail in section [6019] of Reference RF1.

Polymeric dispersant(s) for inorganic binder compositions is/are defined in more detail in section [6020] of Reference RF1. The inorganic binder composition(s) comprising the polymeric dispersants and their use are defined in more detail in section [6021] of Reference RF1. The converting step(s) to obtain the polymeric dispersant(s) are defined in more detail in section [6020] of Reference RF1. The term "inorganic binder composition" comprising the polymeric dispersant(s), as used in the context of the product Ω herein, comprises preferably in particular hydraulically setting compositions and compositions comprising calcium sulfate and is defined in more detail in section [6021] of Reference RF1 entitled "Inorganic binder compositions comprising the polymeric dispersant and their use". Specific building material formulation(s) comprising polymeric dispersant(s) or building product(s) produced by a building material formulation comprising a polymeric dispersant are disclosed in more detail in section [6021] of Reference RF1.

The term "cosmetic surfactant", as used in the context of the product Ω herein, comprises non-ionic, anionic, cationic and amphoteric surfactants and is defined in more detail in paragraph [7002] of Reference RF1. The term "emollient", as used in the context of the product Ω herein, refers to a chemical compound used for protecting, moisturizing, and/or lubricating the skin and is defined in more detail in paragraph [7003] of Reference RF1. The term "wax", as used in the context of the product Ω herein, comprises pearlizers and opacifiers and is defined in more detail in paragraph [7004] of Reference RF1. The term "cosmetic polymer", as used in the context of the product Ω herein, comprises any polymer that can be used as an ingredient in a cosmetic formulation and is defined in more detail in paragraph [7005] of Reference RF1. The term "UV filter", as used in the context of the product Ω herein, refers to a chemical compound that blocks or absorbs ultraviolet light and is defined in more detail in paragraph [7006] of Reference RF1. The term "further cosmetic ingredient", as used in the context of the product Ω herein, comprises any ingredient suitable for making a cosmetic formulation. Several sources disclose cosmetically acceptable ingredients. E. g. the database Cosing on the internet pages of the European Commission discloses cosmetic ingredients and the International Cosmetic Ingredient Dictionary and Handbook, edited by the Personal Care Products Council (PCPC), discloses cosmetic ingredients. The term "composition and/or formulation thereof' with reference to the cosmetic surfactant, emollient, wax, cosmetic polymer, UV filter and/or further cosmetic ingredient refers to personal care and/or cosmetic compositions or formulations defined in more detail in paragraph [7007] of Reference RF1. The converting step(s) to obtain the cosmetic surfactant, emollient, wax, cosmetic polymer, UV filter or further cosmetic ingredient is/are defined in more detail in paragraph [7008] of Reference RF1.

The terms "polymer B", "polymer composition B", "coating composition", "other functional composition", "foil", "molded body", "coating" and "coated substrate" are well known to the person skilled in the art and are defined in more detail from paragraph [8000] to [8005] of Reference RF1.

The present invention is further illustrated by the following set of embodiments and combinations of embodiments resulting from the dependencies and back-references as indicated. In particular, it is noted that in each instance where a range of embodiments is mentioned, for example in the context of a term such as "The process of any one of embodiments 1 to 4", every embodiment in this range is meant to be explicitly disclosed for the skilled person, i.e. the wording of this term is to be understood by the skilled person as being synonymous to "The process of any one of embodiments 1, 2, 3 and 4". Further, it is explicitly noted that the following set of embodiments represents a suitably structured part of the general description directed to preferred aspects of the present invention, and, thus, suitably supports, but does not represent the claims of the present invention.
1. A process for producing an acid, preferably acetic acid, more preferably bio acetic acid, comprising
   (i) providing a chemical component C comprising one or more of a catalyst, a precursor of the catalyst, a reduced form of the catalyst, and a reduced form of the precursor of the catalyst;
   (ii) preparing a liquid mixture M_{E} comprising at least one alcohol R-CH₂-CH₂-OH with R being H or C₁-C₄-alkyl, a base, and the chemical component C provided in (i);
   (iii) subjecting the liquid mixture M_{E} prepared in (ii) to alcohol conversion conditions in a reaction space S_{R}, obtaining in said reaction space a reaction mixture M_{G} comprising at least one alcohol R-CH₂-CH₂-(CHR-CH₂)ₓ-OH with x being an integer in the range of from 1 to 4 and further comprising unreacted alcohol R-CH₂-CH₂-OH and at least one ester R-CH₂-C(O)-O-R' with R being C₁-C₄-alkyl;
   (iv) separating at least part of the at least one alcohol R-CH₂-CH₂-OH from the reaction mixture M_{G} obtained in (iii), obtaining a mixture M_{GS} depleted in the at least one alcohol R-CH₂-CH₂-OH and comprising the at least one alcohol R-CH₂-CH₂-(CHR-CH₂)ₓ-OH and at least one ester R-CH₂-C(O)-O-R';
   (v) preparing a mixture M_{GB} comprising an aqueous phase P_{A} and an organic phase Po, comprising admixing water with the mixture M_{GS} obtained in (iv), said aqueous phase P_{A} comprising at least part of the at least one ester R-CH₂-C(O)-O-R' and said organic phase P_{O} comprising at least part of the at least one alcohol R-CH₂-CH₂-(CHR-CH₂)ₓ-OH;
   (vi) subjecting the mixture M_{GB} prepared in (v) to phase separation conditions, obtaining an aqueous mixture M_{A} comprising at least part of the at least one ester R-CH₂-C(O)-O-R' and a mixture M_{AC} comprising at least part of the at least one alcohol R-CH₂-CH₂-(CHR-CH₂)ₓ-OH;
   (vii) adding an acid component A to the aqueous mixture M_{A} obtained in (vi), obtaining an aqueous mixture M_{AA} comprising an acid R-CH₂-C(O)OH derived from the ester R-CH₂-C(O)-O-R';
   (viii) separating at least part of the acid R-CH₂-C(O)OH from the mixture M_{AA} obtained in (vii)
   wherein
   the acid component A comprises an acid selected from then group consisting of sulfuric acid, nitric acid, citric acid, phosphoric acid, boric acid, carbonic acid, and a mixture of two or more thereof;
   the base is selected from the group consisting of alkali hydroxides, alkaline earth hydroxides, alkali carbonates, alkali hydrogen carbonates, alkaline earth carbonates, alkaline hydrogen carbonates, alkali alkoxides, alkaline earth alkoxides, alkali metal amides, alkaline earth metal amides, alkali metal diisopropylamides, alkaline earth metal diisopropylamides, secondary amino acids, and a mixture of two or more thereof;
   wherein the catalyst comprises a compound of formula (A) 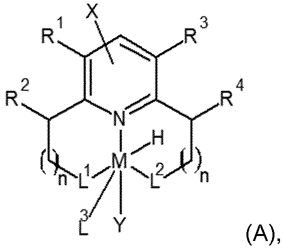 wherein
      M is selected from the group consisting of Ir, Mn, Os, Pd, Pt, Rh, and Ru;
      L¹ and L² are, independently of each other, PR^{a}R^{b}, NR^{a}R^{b}, SR^{a}, SH, S(=O)R^{a}, heteroaryl containing at least one heteroatom selected from nitrogen and sulfur, AsR^{a}R^{b}, SbR^{a}R^{b}, and a N-heterocyclic carbene represented by the structures: 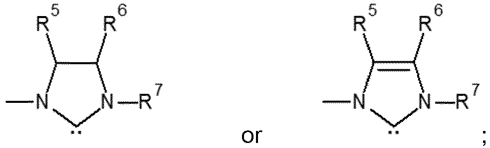
      L³ is selected from the group consisting of CO, PR^{a}R^{b}R^{c}, AsR^{a}R^{b}R^{c}, SbR^{a}R^{b}R^{c}, SR^{a}R^{b}, R^{d}CN, R^{d}NC, N₂, PF₃, pyridine, and thiophene;
      R¹, R², R³ and R⁴ either are hydrogen, or form together with the pyridyl unit of the catalyst of formula (A) an acridinyl unit, or R¹ and R² or R³ and R⁴ form together with the pyridyl unit of the catalyst of formula (A) a quinolinyl unit;
      n is 0 or 1;
      Y is selected from the group consisting of H, F, Cl, Br, I, OC(=O)CF₃, OSO₂CF₃, CN, CO, OH, OR, NR^{d}₂, NH₃, NR^{d}₃, and R^{d}₂NSO₂R^{d};
      R^{a}, R^{b}, R^{c}, R^{d}, R⁵, R⁶ and R⁷ are, independently of each other, selected from the group consisting of H, unsubstituted or substituted C₁-C₁₀-alkyl, wherein the substituents are selected from the group consisting of F, Cl, Br, OH, CN, NH₂, and C₁-C₁₀-alkyl; unsubstituted or substituted C₁-C₁₀-cycloalkyl, wherein the substituents are selected from the group consisting of F, Cl, Br, OH, CN, NH₂, and C₁-C₁₀-alkyl; unsubstituted or substituted C₃-C₁₀-heterocyclyl comprising at least one heteroatom selected from the group consisting of N, O, and S, wherein the substituents are selected from the group consisting of F, Cl, Br, OH, CN, NH₂, and C₁-C₁₀-alkyl; unsubstituted or substituted C₅-C₁₀-aryl, wherein the substituents are selected from the group consisting of F, Cl, Br, OH, CN, NH₂, and C₁-C₁₀-alkyl; and unsubstituted or substituted C₅-C₁₀-heteroaryl comprising at least one heteroatom selected from the group consisting of N, O, and S, wherein the substituents are selected from the group consisting of F, Cl, Br, OH, CN, NH₂ and C₁-C₁₀-alkyl; and
      X is selected from the group consisting of one, two, three, four, five, six, and seven substituents positioned at any carbon atom on the acridinyl unit, or one, two, three, four and five substituents positioned at any carbon atom on the quinolinyl unit, or one substituent positioned at the carbon atom on the pyridyl unit, wherein the substituents are selected from the group consisting of F, Cl, Br, OH, CN, NH₂, and C₁-C₁₀-alkyl;
      the precursor of the catalyst comprising a compound of formula (A) comprises a mixture comprising a compound comprising a metal M and at least one component selected from the group consisting of CO, PR^{a}R^{b}R^{c}, SR^{a}R^{b}, R^{a}CN, R^{a}NC, N₂, PF₃, organic carbonyl compounds, C₁-C₁₀-alkyl, C₁-C₁₂-cycloalkyl, C₂-C₁₂-alkenyl, C₃-C₁₅-cycloalkenyl, C₅-C₂₀-aryl, CN, CO, OH, OC(=O)CF₃, OSO₂CF₃, hydrides, pyridines, halogenides, hydroxides, and thiophenes; and a compound of formula (H) 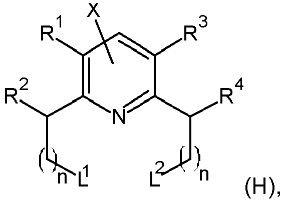
      M is selected from the group consisting of Ir, Mn, Os, Pd, Pt, Rh, and Ru;
      L¹ and L², are, independently of each other, PR^{a}R^{b}, NR^{a}R^{b}, SR^{a}, SH, S(=O)R^{a}, heteroaryl containing at least one heteroatom selected from nitrogen and sulfur, AsR^{a}R^{b}, SbR^{a}R^{b}, and a N-heterocyclic carbene represented by the structures: 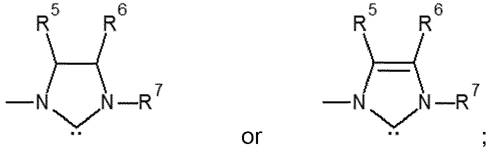
      R¹, R², R³ and R⁴ either are hydrogen, or form together with the pyridyl unit of the catalyst of formula (A) an acridinyl unit, or R¹ and R² or R³ and R⁴ form together with the pyridyl unit of the catalyst of formula (A) a quinolinyl unit;
      n is 0 or 1;
      R^{a}, R^{b}, R^{c}, R^{d}, R⁵, R⁶ and R⁷ are, independently of each other, selected from the group consisting of H, unsubstituted or substituted C₁-C₁₀-alkyl, wherein the substituents are selected from the group consisting of F, Cl, Br, OH, CN, NH₂, and C₁-C₁₀-alkyl; unsubstituted or substituted C₁-C₁₀-cycloalkyl, wherein the substituents are selected from the group consisting of F, Cl, Br, OH, CN, NH₂, and C₁-C₁₀-alkyl; unsubstituted or substituted C₃-C₁₀-heterocyclyl comprising at least one heteroatom selected from the group consisting of N, O, and S, wherein the substituents are selected from the group consisting of F, Cl, Br, OH, CN, NH₂, and C₁-C₁₀-alkyl; unsubstituted or substituted C₅-C₁₀-aryl, wherein the substituents are selected from the group consisting of F, Cl, Br, OH, CN, NH₂, and C₁-C₁₀-alkyl; and unsubstituted or substituted C₅-C₁₀-heteroaryl comprising at least one heteroatom selected from the group consisting of N, O, and S, wherein the substituents are selected from the group consisting of F, Cl, Br, OH, CN, NH₂ and C₁-C₁₀-alkyl;
      X is selected from the group consisting of one, two, three, four, five, six, and seven substituents positioned at any carbon atom on the acridinyl unit, or one, two, three, four and five substituents positioned at any carbon atom on the quinolinyl unit, or one substituent positioned at the carbon atom on the pyridyl unit, wherein the substituents are selected from the group consisting of F, Cl, Br, OH, CN, NH₂, and C₁-C₁₀-alkyl.
2. The process of embodiment 1, wherein the acid component A comprises an acid selected from the group consisting of sulfuric acid, nitric acid, phosphoric acid, boric acid, and a mixture of two or more thereof, preferably wherein the acid component A comprises nitric acid, phosphoric acid or sulfuric acid, more preferably wherein the acid component A comprises sulfuric acid.
3. The process of embodiment 1 or 2, wherein (vii) comprises adding the acid component A to a pH of the aqueous mixture M_{A} in the range of from 1 to 5, preferably of from 1 to 4, more preferably of from 1 to 3.
4. The process of any one of embodiments 1 to 3, wherein separating at least part of the acid R-CH₂-C(O)OH from the mixture M_{AA} in (viii) comprises stripping, distillation or membrane separation.
5. The process of any one of embodiments 1 to 4, wherein R is H, integer x is 1 and the acid R-CH₂-C(O)OH is acetic acid.
6. The process of any one of embodiments 1 to 5, wherein the amount of the at least one ester R-CH₂-C(O)-O-R' prepared in the reaction mixture M_{G} in (iii) is in a range of from 1 to 10 mol%, preferably 1 to 8%, more preferably 2 to 8 mol%, based on 100 mol% of the at least one alcohol R-CH₂-CH₂-OH.
7. The process of any one of embodiments 1 to 6, wherein the base is selected from the group consisting of alkali hydroxides, alkali alkoxides, and a mixture thereof.
8. The process of embodiment 7, wherein the alkali hydroxide is selected from the group consisting of NaOH, KOH, and a mixture thereof, preferably wherein the alkali hydroxide is KOH.
9. The process of embodiment 7, wherein the alkali alkoxide is selected from the group consisting of sodium alkoxides, potassium alkoxides, and a mixture thereof, preferably from the group consisting of sodium ethoxide, potassium ethoxide, and a mixture thereof.
10. The process of any one of embodiments 1 to 9, wherein integer x is 1 or 2, preferably wherein integer x is 1.
11. The process of any one of embodiments 1 to 10, wherein the aqueous phase P_{A} obtained in (v) comprises from 50 to 100 % of the at least one ester R-CH₂-C(O)-O-R', preferably from 60 to 100 % of the at least one ester R-CH₂-C(O)-O-R', more preferably from 70 to 100 % of the at least one ester R-CH₂-C(O)-O-R', more preferably from 80 to 100 % of the at least one ester R-CH₂-C(O)-O-R', based on 100 % of the ester R-CH₂-C(O)-O-R' content in in the mixture M_{GS}.
12. The process of any one of embodiments 1 to 11, further comprising (ix) recycling at least a part of the separated at least one alcohol R-CH₂-CH₂-OH from the reaction mixture M_{G} obtained in (iv) to the liquid mixture M_{E} in (ii) or in (iii).
13. The process of any one of embodiments 1 to 12, further comprising (x) separating at least part of the at least one alcohol R-CH₂-CH₂-(CHR-CH₂)ₓ-OH from the mixture M_{AC} obtaining a mixture M_{ACS} depleted in the at least one alcohol R-CH₂-CH₂-(CHR-CH₂)ₓ-OH.
14. The process of embodiment 13, wherein separating at least part of the at least one alcohol R-CH₂-CH₂-(CHR-CH₂)ₓ-OH in step (x) comprises subjecting the mixture M_{AC} to distillation.
15. The process of embodiment 14, wherein the distillation conditions comprise a temperature in the range of from 70 to 180 °C, preferably from 80 to 160 °C, more preferably from 90 to 150 °C, more preferably from 100 to 140 °C.
16. The process of embodiment 14 or 15, wherein the distillation conditions comprise a pressure in the range of from 1 x 10³ to 2 x 10⁵ Pa, preferably from 2 x 10³ to 1.7 x 10⁵ Pa more preferably from 3 x 10³ to 1.4 x 10⁵ Pa, more preferably from 5 x 10³ to 1 x 10⁵ Pa.
17. The process of any one of embodiments 1 to 16, wherein the alcohol conversion conditions in (iii) comprise a temperature of the reaction mixture M_{G} in the range of from 100 to 250 °C and a pressure in the reaction space S_{G} in the range of from 1 x 10⁵ to 4 x 10⁶ Pa.
18. The process of any one of embodiments 1 to 17, wherein the alcohol conversion conditions according to (iii) comprise a temperature of the reaction mixture M_{G} in the range of from 100 to 200 °C, preferably in the range of from 120 to 180 °C, more preferably in the range of from 120 to 170 °C, more preferably in the range of from 140 to 170 °C.
19. The process of any one of embodiments 1 to 18, wherein the process is a continuous process.
20. The process of any one of embodiments 1 to 18, wherein the process is a semi-batch process or a batch process.
21. The process of any one of embodiments 1 to 20, wherein the alcohol conversion conditions according to (iii) comprise the presence of at least one inert gas in the reaction space S_{R}, wherein the at least one inert gas is preferably selected from the group consisting of nitrogen, argon, and a mixture thereof.
22. The process of any one of embodiments 1 to 21, wherein the alcohol conversion conditions according to (iii) comprise a pressure in the reaction space S_{R} in the range of from 1 x 10⁵ to 3.5 x 10⁶ Pa, preferably in the range of from 1 x 10⁵ to 3.1 x 10⁶ Pa, more preferably in the range in the range of from 1 x 10⁵ to 2 x 10⁶ Pa, more preferably in the range in the range from 1 x 10⁵ to 1.5 x 10⁶ Pa.
23. The process of any one of embodiments 1 to 22, wherein in (iii), the reaction space S_{R} comprises a gas phase, wherein said gas phase comprises H₂, preferably wherein the H₂ partial pressure of the gas phase in the reaction space S_{G} is maintained in the range of from 2 x 10⁴ to 3.1 x 10⁶ Pa, preferably in the range of from 2 x 10⁴ to 1.1 x 10⁶ Pa, more preferably in the range of from 2 x 10⁴ to 6 x 10⁵ Pa.
24. The process of embodiment 23, wherein the H₂ partial pressure of the gas phase is maintained by relaxation of the gas phase or by introducing H₂ into the gas phase.
25. The process embodiment 23 or 24, wherein the H₂ partial pressure of the gas phase is maintained by relaxation of the gas phase.
26. The process of any one of embodiments 1 to 25, wherein the liquid mixture M_{E} prepared according to (ii) further comprises a solvent component S.
27. The process of embodiment 26, wherein the solvent component S comprises a solvent which has a boiling point of 110 °C or more, preferably a boiling point of 140 °C or more, more preferably a boiling point of 160 °C or more, more preferably a boiling point of 180 °C or more, more preferably a boiling point of 190 °C or more.
28. The process of embodiment 26 or 27, wherein the solvent in the solvent component S has a solubility in water at 25 °C of from 0 to 0.7 weight-%, preferably a solubility in water at 25 °C of from 0 to 0.5 weight-%, more preferably a solubility in water at 25 °C of from 0 to 0.1 weight-%, more preferably a solubility in water at 25 °C of from 0 to 0.05 weight-%.
29. The process of any one of embodiments 26 to 28, wherein a distribution coefficient of the catalyst in a system of the solvent component S and water is from 0 to 0.01, preferably from 0 to 0.005, more preferably from 0 to 0.005, based on 1 kg catalyst.
30. The process of any one of embodiments 26 to 29, wherein the solvent component S comprises a mixture of at least two solvents with a boiling point of 140 °C or more, preferably with a boiling point of 160 °C or more, more preferably with a boiling point of 180 °C or more, more preferably with a boiling point of 190 °C or more.
31. The process of embodiments 26 to 30, wherein the solvent component S comprises a solvent which is selected from the group consisting of biphenyl, diphenyl ether, 1-tert-butyl-3,5-dimethyl-benzene, ethylbenzene, cyclododecane, cyclononane, cyclooctane, cycloheptane, decaline, n-butylbutyrate, n-hexylhexyrate, n-octyloctyrate, texanole, di-n-butylether, di-iso-butylether, di-sec-butylether, 1-hexanol, 1-octanol, 1-decanol, 1-dodedacanol, 2-ethylbutan-1-ol, 2-ethylhexan-1-ol, 2-ethyloctan-1-ol, 2-ethyldecan-1-ol, 2-ethyldodecan-1-ol, 2-butylhexan-1-ol, 2-butyloctan-1-ol, 2-butyldecan-1-ol, 2-butyldodecan-1-ol, 2-hexyldecanol, 2-octyldodecanol, 2-propylheptan-1-ol, and a mixture of two or more thereof; preferably wherein the solvent component S comprises at least one solvent selected from the group consisting of 2-ethylbutan-1-ol, 2-ethylhexan-1-ol, 2-ethyloctan-1-ol, 2-ethyldecan-1-ol, 2-ethyldodecan-1-ol, 2-butylhexan-1-ol, 2-butyloctan-1-ol, 2-butyldecan-1-ol, 2-butyldodecan-1-ol, 2-hexadecanol, 2-octyldodecanol, 2-propylheptan-1-ol, and a mixture of two or more thereof.
32. The process of any one of embodiments 26 to 31, wherein the solvent component S does not include any one of benzene, toluene, xylene or mesitylene.
33. The process of any one of embodiments 26 to 32, wherein the alcohol conversion conditions according to (iii) comprise an amount of the solvent component S in the reaction mixture M_{G} in the range of from 5 to 50 weight-%, preferably in the range of from 5 to 30 weight-%, more preferably in the range of from 5 to 10 weight-%, based on the total weight of the reaction mixture M_{G}.
34. The process of any one of embodiments 26 to 33, wherein from 90 to 100 weight-%, preferably from 95 to 100 weight-%, more preferably from 98 to 100 weight-%, more preferably from 99 to 100 weight-% of the liquid mixture M_{E} prepared according to (ii) consist of the at least one alcohol R-CH₂-CH₂-OH, the base, the solvent component S and the chemical component C.
35. The process of any one of embodiments 26 to 34, wherein the mixture M_{ACS} obtained according to (x) further comprises at least part of the chemical component C, and preferably further comprises at least part of the solvent component S.
36. The process of embodiment 35, further comprising
   (xi) recycling at least a part of the solvent component S comprised in the mixture M_{ACS} obtained according to (x) to (ii) or (iii).
37. The process of embodiment 36, further comprising
   (xii) recycling at least a part of the solvent component S and at least a part of the chemical component C comprised in the mixture M_{ACS} obtained according to (x) to (ii) or (iii).
38. The process of any one of embodiments 26 to 37, wherein the solvent does not form an azeotrope with water.
39. The process of any one of embodiments 1 to 38, wherein in formula (A) n is 0 if R¹, R², R³ and R⁴ are hydrogen.
40. The process of any one of embodiments 1 to 39, wherein the reaction mixture M_{G} in (iii) further comprises water; preferably wherein the amount of water in reaction mixture M_{G} is 0.2 weight-% or less, more preferably in the range of from 0 to 0.2 weight-%, more preferably from 0.0001 to 0.2 weight-%, more preferably from 0.0001 to 0.15 weight-%, more preferably from 0.0005 to 0.1 weight-%, more preferably from 0.0005 to 0.08 weight-%, more preferably from 0.0005 to 0.05 weight-%, based on the total weight-% of the reaction mixture M_{G}.
41. The process of embodiment 40, wherein step (iii) further comprises at least partially removing of water from the reaction mixture reaction mixture M_{G}, preferably the continuous removal of at least a part of water from the reaction mixture M_{G}.
42. The process of any one of embodiments 1 to 41, wherein the chemical component C comprises a compound of formula (B) 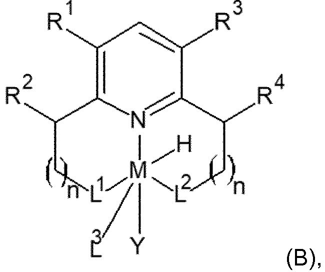 wherein
   M is selected from the group consisting of Ir, Mn, Os, Pd, Pt, Rh, and Ru;
   L¹ and L² are, independently of each other, PR^{a}R^{b}, NR^{a}R^{b}, SR^{a}, SH, and S(=O)R^{a};
   L³ is selected from the group consisting of CO, PR^{a}R^{b}R^{c}, SR^{a}R^{b}, R^{a}CN, R^{a}NC, N₂, PF₃, pyridine, and thiophene;
   R¹, R², R³ and R⁴ either are hydrogen, or form together with the pyridyl unit of the catalyst of formula (A) an acridinyl unit;
   n is 0 or 1, and if R¹, R², R³ and R⁴ are hydrogen, n is 0;
   R^{a}, R^{b}, R^{c} and R^{d} are, independently of each other, selected from the group consisting of H, unsubstituted or substituted C₁-C₁₀-alkyl wherein the substituents are selected from the group consisting of F, Cl, Br, OH, CN, NH₂, and C₁-C₁₀-alkyl; unsubstituted or substituted C₁-C₁₀-cycloalkyl wherein the substituents are selected from the group consisting of F, Cl, Br, OH, CN, NH₂, and C₁-C₁₀-alkyl; C₃-C₁₀-heterocyclyl comprising at least one heteroatom selected from the group consisting of N, O, and S; C₅-C₁₀-aryl; and C₅-C₁₀-heteroaryl comprising at least one heteroatom selected from the group consisting of N, O, and S;
   Y is selected from the group consisting of H, F, Cl, Br, I, OC(=O)CF₃, OSO₂CF₃, CN, CO, and OH;
   and wherein for the compound of formula (L), R¹, R², R³ and R⁴, L¹, L² and n are preferably identical to R¹, R², R³ and R⁴, L¹, L² and n of the catalyst of formula (B).
43. The process of any one of embodiments 1 to 41, wherein the chemical component C comprises a compound of formula (C) 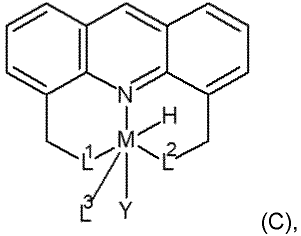 wherein
   M is selected from the group consisting of Ir, Ru, and Mn;
   L¹ and L² are, independently of each other, PR^{a}R^{b}, NR^{a}R^{b}, SR^{a}, SH, and S(=O)R^{a};
   L³ is selected from the group consisting of CO, PR^{a}R^{b}R^{c}, SR^{a}R^{b}, R^{a}CN, R^{a}NC, N₂, PF₃, pyridine, and thiophene;
   R^{a}, R^{b}, R^{c} and R^{d} are, independently of each other, selected from the group consisting of H, unsubstituted or substituted C₁-C₁₀ alkyl wherein the substituents are selected from the group consisting of F, Cl, Br, OH, CN, NH₂, and C₁-C₁₀ alkyl; unsubstituted or substituted C₁-C₁₀-cycloalkyl wherein the substituents are selected from the group consisting of F, Cl, Br, OH, CN, NH₂, and C₁-C₁₀ alkyl; C₃-C₁₀ heterocyclyl comprising at least one heteroatom selected from the group consisting of N, O, and S; C₅-C₁₀ aryl; and C₅-C₁₀ heteroaryl comprising at least one heteroatom selected from the group consisting of N, O, and S; Y is selected from the group consisting of H, F, Cl, Br, I, OC(=O)CF₃, OSO₂CF₃, CN, CO, and OH;
   and wherein for the compound of formula (L), R¹, R², R³ and R⁴, L¹, L², and n are preferably identical to R¹, R², R³ and R⁴, L¹, L², and n of the catalyst of formula (C).
44. The process of any one of embodiments 1 to 41, wherein the chemical component C comprises a compound of formula (D) 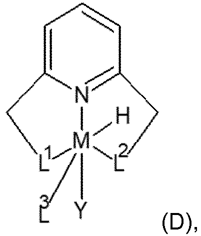 wherein
   M is selected from the group consisting of Ir, Ru, and Mn;
   L¹ and L² are, independently of each other, PR^{a}R^{b}, NR^{a}R^{b}, SR^{a}, SH, and S(=O)R^{a};
   L³ is selected from the group consisting of CO, PR^{a}R^{b}R^{c}, SR^{a}R^{b}, R^{a}CN, R^{a}NC, N₂, PF₃, pyridine, and thiophene;
   R^{a}, R^{b}, R^{c} and R^{d} are, independently of each other, selected from the group consisting of H, unsubstituted or substituted C₁-C₁₀-alkyl wherein the substituents are selected from the group consisting of F, Cl, Br, OH, CN, NH₂, and C₁-C₁₀-alkyl; unsubstituted or substituted C₁-C₁₀-cycloalkyl wherein the substituents are selected from the group consisting of F, Cl, Br, OH, CN, NH₂, and C₁-C₁₀-alkyl; C₃-C₁₀-heterocyclyl comprising at least one heteroatom selected from the group consisting of N, O, and S; C₅-C₁₀ aryl; and C₅-C₁₀-heteroaryl comprising at least one heteroatom selected from the group consisting of N, O, and S;
   Y is selected from the group consisting of H, F, Cl, Br, I, OC(=O)CF₃, OSO₂CF₃, CN, CO, and OH;
   and wherein for the compound of formula (L), R¹, R², R³ and R⁴, L¹, L² and n are preferably identical to R¹, R², R³ and R⁴, L¹, L² and n of the catalyst of formula (D).
45. The process of any one of embodiments 1 to 44, wherein M is selected from the group consisting of Ir and Ru, preferably wherein M is Ru.
46. The process of any one of embodiments 1 to 45, wherein L³ is CO.
47. The process of any one of embodiments 1 to 45, wherein L¹ and L² are each (PR^{a}R^{b}), and wherein R^{a} and R^{b} are C₁-C₁₀-alkyl, preferably wherein R^{a} and R^{b} are each isopropyl or tert-butyl.
48. The process of any one of embodiments 1 to 45, wherein L¹ and L² are each (PR^{a}R^{b}), and wherein R^{a} and R^{b} are C₁-C₁₀-cycloalkyl, preferably wherein R^{a} and R^{b} are each cyclohexyl.
49. The process of any one of embodiments 1 to 45, wherein L¹ and L² are each (PR^{a}R^{b}), and wherein R^{a} and R^{b} are C₅-C₁₀-aryl.
50. The process of any one of embodiments 1 to 49, wherein Y is selected from the group consisting of F, Cl, Br and I, preferably wherein Y is selected from the group consisting of Cl or Br, more preferably wherein Y is Cl.
51. The process of any one of embodiments 1 to 49, wherein Y is CO.
52. The process of any one of embodiments 1 to 41, wherein the chemical component C comprises a compound of formula (E) 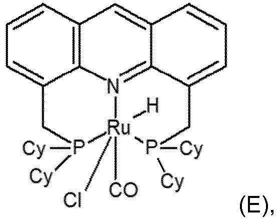 wherein Cy is cyclohexyl.
53. The process of any one of embodiments 1 to 41, wherein the reduced form of the catalyst comprises a compound of formula (E') 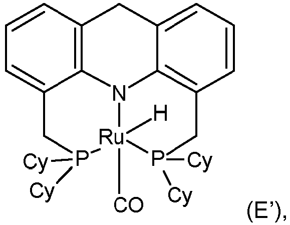 wherein Cy is cyclohexyl.
54. The process of any one of embodiments 1 to 41, wherein the chemical component C comprises a compound of formula (F) 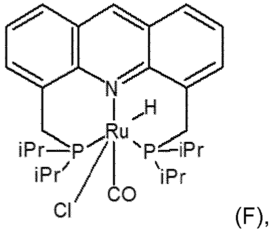 wherein iPr is isopropyl.
55. The process of any one of embodiments 1 to 41, wherein the reduced form of the catalyst comprises a compound of formula (F') 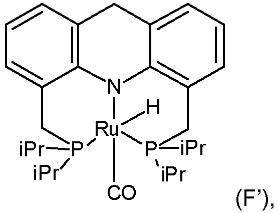 wherein iPr is isopropyl.
56. The process of any one of embodiments 1 to 41, wherein the chemical component C comprises a compound of formula (G) 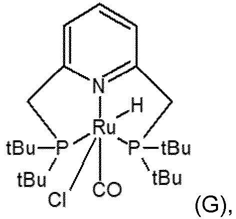 wherein tBu is tert-butyl.
57. The process of any one of embodiments 1 to 56, wherein the reduced form of the catalyst comprises a compound of formula (G') 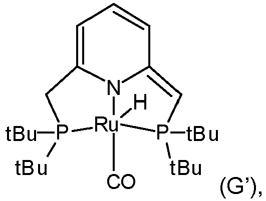 wherein tBu is tert-butyl.
58. The process of any one of embodiments 1 to 57, wherein the chemical component C comprises a compound comprising a metal M selected from the group consisting of IrCl₃ x HzO, [Ir(COD)Cl]₂, [Ir(COE)zCl]z, [Ir(C₂H₄)₂Cl]₂, [Ir(COD)OH]₂, [Ir(COD)MeO]₂, [IrCp*Cl₂], [IrCp Cl₂], Ir₄(CO)₁₂, [Ir(PPh₃)₂(CO)Cl], [Ir(acetylacetonate)₃], and [lr(acetylacetonate)(COD)], wherein Cp is cylclopentadienyl, Cp* is pentamethylcyclopentadienyl, COD is 1,5-cyclooctadienyl, COE is cyclooctenyl, and methylallyl is 2-methylallyl.
59. The process of any one of embodiments 1 to 58, wherein the chemical component C comprises a compound comprising a metal M selected from the group consisting of [Ru(p-cymene)Cl₂]₂, [Ru(benzene)Cl₂]_{y}, [Ru(CO)₂Cl₂]_{y}, where y is in each case in the range from 1 to 1000, [Ru(CO)₃Cl₂]₂, [Ru(COD)(allyl)₂], RuCl₃ x H₂O, [Ru(acetylacetonate)₃], [Ru(DMSO)₄Cl₂], [Ru(cyclopentadienyl)(CO)₂Cl], [Ru(cyclopentadienyl)(CO)₂H], [Ru(cyclopentadienyl)(CO)₂]₂, [Ru(Cp)(CO)₂Cl], [Ru(Cp*)(CO)₂H], [Ru(Cp*)(CO)₂]₂, [Ru(indenyl)(CO)zCl], [Ru(indenyl)(CO)₂H], [Ru(indenyl)(CO)₂]₂, ruthenocene, [Ru(COD)Cl₂]₂, [Ru(Cp*)(COD)CI], [Ru₃(CO)₁₂], [Ru(PPh₃)₄(H)₂], [Ru(PPh₃)₃(Cl)₂], [Ru(PPh₃)₃(CO)(Cl)₂], [Ru(PPh₃)₃(CO)(Cl)(H)], [Ru(PPh₃)₃(CO)(H)₂], and [Ru(cyclooctadienyl)(methylallyl)₂], wherein Cp is cylclopentadienyl, Cp* is pentamethylcyclopentadienyl, COD is 1,5-cyclooctadienyl, and methylallyl is 2-methylallyl.
60. The process of any one of embodiments 1 to 59, wherein the reduced form of the precursor comprises a compound of formula (P-I) or (P-II): 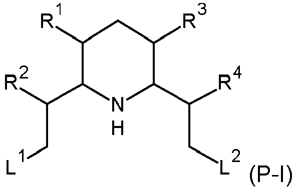
   wherein R¹, R², R³ and R⁴ either are hydrogen, or form together with the N-containing ring a tetrahydroquinoline unit, a decahydroquinoline unit, a tetrahydroacridine unit, or a tetradecahydroacridine unit; and
   wherein L¹ and L² are, independently of each other, as defined above; 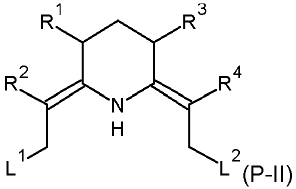
   wherein R¹, R², R³ and R⁴ are hydrogen; and
   wherein L¹ and L² are, independently of each other, as defined above.
61. The process of any one of embodiments 1 to 59, wherein the reduced form of the precursor comprises a compound of formula (P-I): 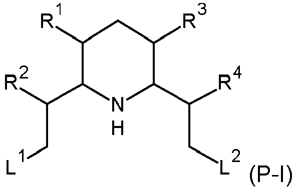 wherein R¹, R², R³ and R⁴ either are hydrogen, or form together with the N-containing ring a tetrahydroacridine unit, or a tetradecahydroacridine unit.
62. The process of any one of embodiments 1 to 59, wherein the reduced form of the precursor comprises a compound of formula (P-II): 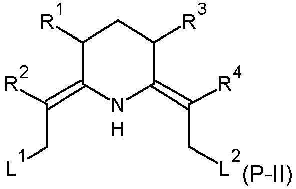
   wherein R¹, R², R³ and R⁴ are hydrogen; and
   wherein L¹ and L² are, independently of each other, as defined above.
63. The process of any one of embodiments 1 to 62, wherein R is selected from the group consisting of H, methyl, ethyl, propyl, isopropyl, butyl, isobutyl, and tert-butyl, preferably from the group consisting of H, methyl, ethyl, propyl, and isopropyl, more preferably from the group consisting of H, ethyl, and propyl, wherein more preferably R is H.
64. The process of any one of embodiments 1 to 63, wherein the liquid mixture M_{E} prepared according to (ii) further comprises a compound of formula (H): 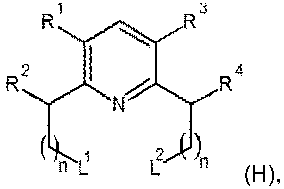 wherein R¹, R², R³ and R^{4,} L¹, L², and n are identical to R¹, R², R³ and R^{4,} L¹, L², and n of the catalyst of formula (A).
65. The process of embodiment 64, wherein in the liquid mixture M_{E} prepared according to (ii) and subjected to alcohol version conditions according to (iii), the molar ratio of the compound of formula (H) relative to the compound of formula (A) is in a range of from 0.01:1 to 10:1, preferably in the range of from 0.05:1 to 10:1, more preferably in the range of from 0.1:1 to 10:1, more preferably in the range of from 0.1:1 to 10:1, more preferably in the range of from 0.3:1 to 10:1, more preferably in the range of from 0.5:1 to 10:1, more preferably in the range of from 0.7:1 to 10:1, more preferably in the range of from 0.8:1 to 10:1, more preferably in the range of from 1:1 to 10:1 more preferably in the range of from 1.01:1 to 10:1, more preferably in the range of from 1.02:1 to 8:1, more preferably in the range from 1.03:1 to 7:1, more preferably in the range from 1.04:1 to 6:1, and more preferably in the range from 1.05:1 to 5:1.
66. The process of embodiment 64 or 65, wherein the compound of formula (H) is selected from the group consisting of dicyclohexyl-[[5-(dicyclohexylphosphanylmethyl)acridin-4-yl]methyl]phosphane, diisopropyl-[[5-(diisopropylphosphanylmethyl)acridin-4-yl]methyl]phosphane, dicyclohexyl-[[5-(dicyclohexylphosphanylmethyl)pyridin-4-yl]methyl]phosphane and diisopropyl-[[5-(diisopropylphosphanylmethyl)pyridin-4-yl]methyl]phosphane, preferably wherein the compound of formula (H) is cyclohexyl-[[5-(dicyclohexylphosphanylmethyl)acridin-4-yl]methyl]phosphane or diisopropyl-[[5-(diisopropylphosphanylmethyl)acridin-4-yl]methyl]phosphane.
67. The process of any one of embodiments 1 to 66, wherein the at least one alcohol R-CH₂-CH₂-OH, is a bio-based alcohol, preferably obtainable or obtained from sugar-containing crops, more preferably from one or more of sugar cane and corn.
68. The process of any one of embodiments 1 to 67, wherein the reaction space S_{R} is comprised in a reactor vessel, wherein the reactor vessel is preferably a complete-mixing reactor vessel.
69. A process, preferably according to any one of embodiments 1 to 68, comprising the step of converting a chemical material obtainable by or obtained by the process according to any one of embodiments 1 to 68 to obtain a product product Ω.
70. The process of embodiment 69, wherein the product Ω is selected from:
   - building block or monomer; or
   - polymer, preferably polymer A, polymer composition, preferably polymer composition A, or polymer product, preferably polymer product A; or
   - industrial use polymer, industrial use surfactant, descaling compound, industrial use biocide, industrial use solvent, industrial use dispersant, composition thereof or formulation thereof; or
   - agrochemical composition, agrochemical formulation auxiliary or agrochemically active ingredient; or
   - active pharmaceutical ingredient or intermediate thereof, pharmaceutical excipient, animal feed additive, human food additive, dietary supplements, aroma chemical or aroma composition; or
   - aqueous polymer dispersion, preferably polyurethane or polyurethane - poly(meth)acrylate hybrid polymer dispersion, emulsion, binder for paper and fiber coatings, UV-curable acrylic polymer for hot melts and coatings polyisocyanates, hyperbranched polyester polyol, polymeric dispersant for inorganic binder compositions, unsaturated polyester polyol or 100% curable composition; or
   - cosmetic surfactant, emollient, wax, cosmetic polymer, UV filter, further cosmetic ingredient or composition or formulation thereof; or
   - polymer B, polymer composition B, coating composition, other functional composition, foil, molded body, coating or coated substrate.
71. The process of embodiment 70,
   wherein the content of the chemical material in the product Ω is 1 weight-% or more, preferably 2 weight-% or more, more preferably 5 weight-% or more, more preferably 15 weight-% or more, more preferably 30 weight-% or more, more preferably 40 weight-% or more, more preferably 60 weight-% or more, more preferably 80 weight-% or more, more preferably 90 weight-% or more, more preferably 95 weight-% or more; and/or
   wherein the content of the chemical material in the product Ω is 100 weight-% or less, preferably 95 weight-% or less, more preferably 90 weight-% or less, more preferably 50 weight-% or less, more preferably 25 weight-% or less, more preferably 10 weight-% or less; and preferably wherein the content is determined based on identity preservation and/or segregation and/or mass balance and/or book and claim chain of custody models, preferably based on mass balance, preferably the International Sustainability and Carbon Certification (ISCC) standard.

The determination of the distribution coefficient of the solvent in water comprises the following steps:
1. combining the two components, e.g. feed and solvent, in a predefined solvent ratio;
2. turbulent mixing of the combined components over a longer period of time (> 10 min) at a defined extraction temperature;
3. allowing for phase separation;
4. taking samples of each phase at the extraction temperature;
5. centrifuging the samples and withdrawing clear samples at the extraction temperature;
6. analyzing the samples; and
7. comparing the results of extract- and raffinate - calculation of the partition equilibrium/partition coefficient at the selected temperature.

### Cited literature:

- M. Guerbet, C. R. Hebd. Séances Acad. Sci. 1899, 128, p. 511-513

## Claims

1. A process for producing an acid, comprising
(i) providing a chemical component C comprising one or more of a catalyst, a precursor of the catalyst, a reduced form of the catalyst, and a reduced form of the precursor of the catalyst;
(ii) preparing a liquid mixture M_{E} comprising at least one alcohol R-CH₂-CH₂-OH with R being H or C₁-C₄-alkyl, a base, and the chemical component C provided in (i);
(iii) subjecting the liquid mixture M_{E} prepared in (ii) to alcohol conversion conditions in a reaction space S_{R}, obtaining in said reaction space a reaction mixture M_{G} comprising at least one alcohol R-CH₂-CH₂-(CHR-CH₂)ₓ-OH with x being an integer in the range of from 1 to 4 and further comprising unreacted alcohol R-CH₂-CH₂-OH and at least one ester R-CH₂-C(O)-O-R' with R' being C₁-C₄-alkyl;
(iv) separating at least part of the at least one alcohol R-CH₂-CH₂-OH from the reaction mixture M_{G} obtained in (iii), obtaining a mixture M_{GS} depleted in the at least one alcohol R-CH₂-CH₂-OH and comprising the at least one alcohol R-CH₂-CH₂-(CHR-CH₂)ₓ-OH and at least one ester R-CH₂-C(O)-O-R';
(v) preparing a mixture M_{GB} comprising an aqueous phase P_{A} and an organic phase Po, comprising admixing water with the mixture M_{GS} obtained in (iv), said aqueous phase P_{A} comprising at least part of the at least one ester R-CH₂-C(O)-O-R' and said organic phase P_{O} comprising at least part of the at least one alcohol R-CH₂-CH₂-(CHR-CH₂)ₓ-OH;
(vi) subjecting the mixture M_{GB} prepared in (v) to phase separation conditions, obtaining an aqueous mixture M_{A} comprising at least part of the at least one ester R-CH₂-C(O)-O-R' and a mixture M_{AC} comprising at least part of the at least one alcohol R-CH₂-CH₂-(CHR-CH₂)ₓ-OH;
(vii) adding an acid component A to the aqueous mixture M_{A} obtained in (vi), obtaining an aqueous mixture M_{AA} comprising an acid R-CH₂-C(O)OH derived from the ester R-CH₂-C(O)-O-R';
(viii) separating at least part of the acid R-CH₂-C(O)OH from the mixture M_{AA} obtained in (vii)
wherein
the acid component A comprises an acid selected from then group consisting of sulfuric acid, nitric acid, citric acid, phosphoric acid, boric acid, carbonic acid, and a mixture of two or more thereof;
the base is selected from the group consisting of alkali hydroxides, alkaline earth hydroxides, alkali carbonates, alkali hydrogen carbonates, alkaline earth carbonates, alkaline hydrogen carbonates, alkali alkoxides, alkaline earth alkoxides, alkali metal amides, alkaline earth metal amides, alkali metal diisopropylamides, alkaline earth metal diisopropylamides, secondary amino acids, and a mixture of two or more thereof;
wherein the catalyst comprises a compound of formula (A) 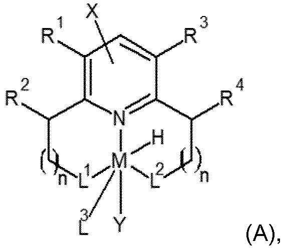 wherein
M is selected from the group consisting of Ir, Mn, Os, Pd, Pt, Rh, and Ru;
L¹ and L² are, independently of each other, PR^{a}R^{b}, NR^{a}R^{b}, SR^{a}, SH, S(=O)R^{a}, heteroaryl containing at least one heteroatom selected from nitrogen and sulfur, AsR^{a}R^{b}, SbR^{a}R^{b}, and a N-heterocyclic carbene represented by the structures: 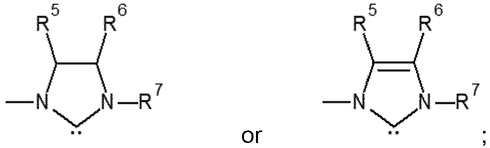
L³ is selected from the group consisting of CO, PR^{a}R^{b}R^{c}, AsR^{a}R^{b}R^{c}, SbR^{a}R^{b}R^{c}, R^{d}CN, SR^{a}R^{b}, R^{d}NC, N₂, PF₃, pyridine, and thiophene;
R¹, R², R³ and R⁴ either are hydrogen, or form together with the pyridyl unit of the catalyst of formula (A) an acridinyl unit, or R' and R² or R³ and R⁴ form together with the pyridyl unit of the catalyst of formula (A) a quinolinyl unit;
n is 0 or 1;
Y is selected from the group consisting of H, F, Cl, Br, I, OC(=O)CF₃, OSO₂CF₃, CN, CO, OH, OR, NR^{d}₂, NH₃, NR^{d}₃, and R^{d}₂NSO₂R^{d};
R^{a}, R^{b}, R^{c}, R^{d}, R⁵, R⁶ and R⁷ are, independently of each other, selected from the group consisting of H, unsubstituted or substituted C₁-C₁₀-alkyl, wherein the substituents are selected from the group consisting of F, Cl, Br, OH, CN, NH₂, and C₁-C₁₀-alkyl;
unsubstituted or substituted C₁-C₁₀-cycloalkyl, wherein the substituents are selected from the group consisting of F, Cl, Br, OH, CN, NH₂, and C₁-C₁₀-alkyl; unsubstituted or substituted C₃-C₁₀-heterocyclyl comprising at least one heteroatom selected from the group consisting of N, O, and S, wherein the substituents are selected from the group consisting of F, Cl, Br, OH, CN, NH₂, and C₁-C₁₀-alkyl; unsubstituted or substituted C₅-C₁₀-aryl, wherein the substituents are selected from the group consisting of F, Cl, Br, OH, CN, NH₂, and C₁-C₁₀-alkyl; and unsubstituted or substituted C₅-C₁₀-heteroaryl comprising at least one heteroatom selected from the group consisting of N, O, and S, wherein the substituents are selected from the group consisting of F, Cl, Br, OH, CN, NH₂ and C₁-C₁₀-alkyl; and
X is selected from the group consisting of one, two, three, four, five, six, and seven substituents positioned at any carbon atom on the acridinyl unit, or one, two, three, four and five substituents positioned at any carbon atom on the quinolinyl unit, or one substituent positioned at the carbon atom on the pyridyl unit, wherein the substituents are selected from the group consisting of F, Cl, Br, OH, CN, NH₂, and C₁-C₁₀-alkyl;
the precursor of the catalyst comprising a compound of formula (A) comprises a mixture comprising a compound comprising a metal M and at least one component selected from the group consisting of CO, PR^{a}R^{b}R^{c}, SR^{a}R^{b}, R^{a}CN, R^{a}NC, N₂, PF₃, organic carbonyl compounds, C₁-C₁₀-alkyl, C₁-C₁₂-cycloalkyl, C₂-C₁₂-alkenyl, C₃-C₁₅-cycloalkenyl, C₅-C₂₀-aryl, CN, CO, OH, OC(=O)CF₃, OSO₂CF₃, hydrides, pyridines, halogenides, hydroxides, and thiophenes; and a compound of formula (H) 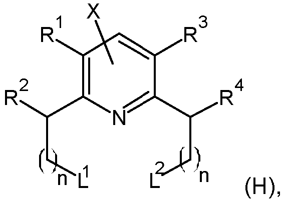
M is selected from the group consisting of Ir, Mn, Os, Pd, Pt, Rh, and Ru;
L¹ and L², are, independently of each other, PR^{a}R^{b}, NR^{a}R^{b}, SR^{a}, SH, S(=O)R^{a}, heteroaryl containing at least one heteroatom selected from nitrogen and sulfur, AsR^{a}R^{b}, SbR^{a}R^{b}, and a N-heterocyclic carbene represented by the structures: 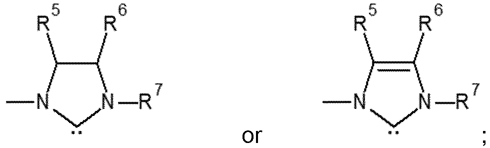
R¹, R², R³ and R⁴ either are hydrogen, or form together with the pyridyl unit of the catalyst of formula (A) an acridinyl unit, or R' and R² or R³ and R⁴ form together with the pyridyl unit of the catalyst of formula (A) a quinolinyl unit;
n is 0 or 1;
R^{a}, R^{b}, R^{c}, R^{d}, R⁵, R⁶ and R⁷ are, independently of each other, selected from the group consisting of H, unsubstituted or substituted C₁-C₁₀-alkyl, wherein the substituents are selected from the group consisting of F, Cl, Br, OH, CN, NH₂, and C₁-C₁₀-alkyl;
unsubstituted or substituted C₁-C₁₀-cycloalkyl, wherein the substituents are selected from the group consisting of F, Cl, Br, OH, CN, NH₂, and C₁-C₁₀-alkyl; unsubstituted or substituted C₃-C₁₀-heterocyclyl comprising at least one heteroatom selected from the group consisting of N, O, and S, wherein the substituents are selected from the group consisting of F, Cl, Br, OH, CN, NH₂, and C₁-C₁₀-alkyl; unsubstituted or substituted C₅-C₁₀-aryl, wherein the substituents are selected from the group consisting of F, Cl, Br, OH, CN, NH₂, and C₁-C₁₀-alkyl; and unsubstituted or substituted C₅-C₁₀-heteroaryl comprising at least one heteroatom selected from the group consisting of N, O, and S, wherein the substituents are selected from the group consisting of F, Cl, Br, OH, CN, NH₂ and C₁-C₁₀-alkyl;
X is selected from the group consisting of one, two, three, four, five, six, and seven substituents positioned at any carbon atom on the acridinyl unit, or one, two, three, four and five substituents positioned at any carbon atom on the quinolinyl unit, or one substituent positioned at the carbon atom on the pyridyl unit, wherein the substituents are selected from the group consisting of F, Cl, Br, OH, CN, NH₂, and C₁-C₁₀-alkyl.

2. The process of claim 1, wherein the acid component A comprises an acid selected from the group consisting of sulfuric acid, nitric acid, phosphoric acid, boric acid, and a mixture of two or more thereof, preferably wherein the acid component A comprises nitric acid, phosphoric acid or sulfuric acid, more preferably wherein the acid component A comprises sulfuric acid.

3. The process of claim 1 or 2, wherein (vii) comprises adding the acid component A to a pH of the aqueous mixture M_{A} in the range of from 1 to 5, more preferably of from 1 to 4, more preferably of from 1 to 3.

4. The process of any one of claims 1 to 3, wherein separating at least part of the acid R-CH₂-C(O)OH from the mixture M_{AA} in (viii) comprises stripping, distillation or membrane separation.

5. The process of any one of claims 1 to 4, wherein R is H, integer x is 1 and the acid R-CH₂-C(O)OH is acetic acid.

6. The process of any one of claims 1 to 5, wherein the amount of the at least one ester R-CH₂-C(O)-O-R' prepared in the reaction mixture M_{G} in (iii) is in a range of from 1 to 10 mol%, preferably 1 to 8%, more preferably 2 to 8 mol%, based on 100 mol% of the at least one alcohol R-CH₂-CH₂-OH.

7. The process of any one of claims 1 to 6, wherein the aqueous phase P_{A} obtained in (v) comprises from 50 to 100 % of the at least one ester R-CH₂-C(O)-O-R', preferably from 60 to 100 % of the at least one ester R-CH₂-C(O)-O-R', more preferably from 70 to 100 % of the at least one ester R-CH₂-C(O)-O-R', more preferably from 80 to 100 % of the at least one ester R-CH₂-C(O)-O-R', based on 100 % of the ester R-CH₂-C(O)-O-R' content in in the mixture M_{GS}.

8. The process of any one of claims 1 to 7, further comprising
(ix) recycling at least a part of the separated at least one alcohol R-CH₂-CH₂-OH from the reaction mixture M_{G} obtained in (iv) to the liquid mixture M_{E} in (ii) or in (iii).

9. The process of any one of claims 1 to 8, further comprising
(x) separating at least part of the at least one alcohol R-CH₂-CH₂-(CHR-CH₂)ₓ-OH from the mixture M_{AC} obtaining a mixture M_{ACS} depleted in the at least one alcohol R-CH₂-CH₂-(CHR-CH₂)ₓ-OH.

10. The process of any one of claims 1 to 9, wherein the liquid mixture M_{E} prepared according to (ii) further comprises a solvent component S.

11. The process of claim 9 or 10, wherein the mixture M_{ACS} obtained according to (x) further comprises at least part of the chemical component C, and preferably further comprises at least part of the solvent component S.

12. The process of claim 11, further comprising
(xi) recycling at least a part of the solvent component S comprised in the mixture M_{ACS} obtained according to (x) to (ii) or (iii).

13. The process of claim 11, further comprising
(xii) recycling at least a part of the solvent component S and at least a part of the chemical component C comprised in the mixture M_{ACS} obtained according to (x) to (ii) or (iii).

14. The process of any one of claims 1 to 13, wherein the at least one alcohol R-CH₂-CH₂-OH, is a bio-based alcohol, preferably obtainable or obtained from sugar-containing crops, more preferably from one or more of sugar cane and corn.

15. A process, preferably according to any one of claims 1 to 14, comprising the step of converting a chemical material obtainable by or obtained by the process according to any one of claims 1 to 14 to obtain a product product Ω.
